(19) Europäisches Patentamt
European Patent Office
Office européen des brevets

(11) **EP 4 183 777 A1**

(12) **EUROPEAN PATENT APPLICATION**
published in accordance with Art. 153(4) EPC

(43) Date of publication:
**24.05.2023 Bulletin 2023/21**

(21) Application number: **20945846.2**

(22) Date of filing: **20.07.2020**

(51) International Patent Classification (IPC):
***C07D 257/04*** *(2006.01)* ***C07D 317/46*** *(2006.01)*
***C07D 405/12*** *(2006.01)* ***C07D 237/04*** *(2006.01)*
***A61K 31/50*** *(2006.01)* ***A61K 31/41*** *(2006.01)*
***A61K 31/36*** *(2006.01)* ***A61P 7/02*** *(2006.01)*

(52) Cooperative Patent Classification (CPC):
**A61K 31/36; A61K 31/41; A61K 31/50; A61P 7/02; C07D 237/04; C07D 257/04; C07D 317/46; C07D 405/12**

(86) International application number:
**PCT/CN2020/103136**

(87) International publication number:
**WO 2022/016345 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Shenzhen Mindray Bio-Medical Electronics Co., Ltd**
**Shenzhen, Guangdong 518057 (CN)**

(72) Inventors:
- **GAO, Fei**
**Shenzhen, Guangdong 518057 (CN)**
- **ZHANG, Ziqian**
**Shenzhen, Guangdong 518057 (CN)**

(74) Representative: **KIPA AB**
**Drottninggatan 11**
**252 21 Helsingborg (SE)**

(54) **APPLICATION OF COMPOSITION FOR PLATELET DISAGGREGATION, DISAGGREGATION REAGENT AND DISAGGREGATION METHOD**

(57) Disclosed is use of a composition for preventing and/or eliminating platelet aggregation in an *in vitro* blood sample. The composition comprises at least one compound selected from the group consisting of formula (I) and a salt thereof. Also disclosed is an agent, which comprises the compound for reducing platelet aggregation interference in an *in vitro* blood test, and a method for preventing and/or eliminating platelet aggregation in a sample in an *in vitro* blood test. The compound of the present invention exhibits a disaggregation effect in multiple types of platelet aggregation circumstances, and the platelet disaggregation takes effect within a short time without additional conditions such as temperature control with water bath, prolonged reaction time and the like, thereby eliminating platelet aggregation in a sample conveniently and thus accurate blood cell detection parameters can be obtained.

R1-NH-R2 (I)

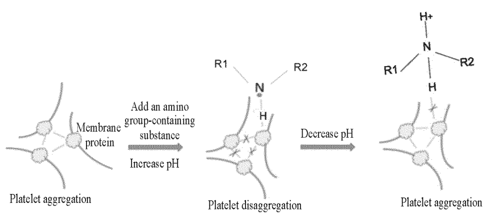


*FIG. 1*

EP 4 183 777 A1

## Description

### TECHNICAL FIELD

The disclosure relates to an *in vitro* blood test, in particular to the use of a compound capable of eliminating platelet aggregation in platelet disaggregation, a agent comprising the compound, and a disaggregation method.

### BACKGROUND

During the analysis of blood cells, platelet pseudo-aggregation results in wrong blood cell count and classification, thereby causing wrong diagnosis and treatment to patients. It is easy to confuse platelet pseudo-aggregation with some life-threatening diseases, such as heparin-induced *in vivo* platelet aggregation (HIP) and disseminated intravascular coagulation (DIC), or make wrong treatment decisions, such as wrong medication, inappropriate platelet transfusion to patients or even splenectomy. There are numerous and complex factors causing platelet pseudo-aggregation. Common factors include: ethylenediaminetetraacetic acid-dependent pseudo-thrombocytopenia (EDTA-PTCP), multiple anticoagulant-dependent platelet aggregation, platelet satellitism, hypercholesterolemia, hypertriglyceridemia, cold-induced platelet aggregation caused by low temperature environment, aggregation caused by unsmooth blood collection and materials of anticoagulant tubes, etc. There are few studies on the mechanism of platelet pseudo-aggregation, most of which focuses on EDTA-PTCP. EDTA is a clinically widely used anticoagulant as approved by the International Committee for Standardization of Hematology (ICSH). Platelet pseudo-aggregation induced by EDTA was first reported in 1969, and its leading cause may be the presence of EDTA-dependent anti-platelet antibodies in the blood of EDTA-PTCP patients. When the blood is mixed with an EDTA anticoagulant *in vitro,* these antibodies can recognize glycoproteins IIb-IIIa (GpIIb-IIIa) on the platelet membrane and lead to the expression of platelet aggregation activating antigens such as granular membrane protein 140 (GMP140, also known as CD62P or P-selectin), type III lysosomal glycoprotein (Gp55, also known as CD63) and thrombin-sensitive protein, so that tyrosine kinase is activated, causing platelet aggregation.

Platelet pseudo-aggregation not only leads to errors in clinical parameters related to platelets, but also influences the accuracy of clinical parameters of other blood cells, particularly white blood cells, bringing adverse consequences to clinical diagnosis and treatment. Therefore, finding and eliminating platelet aggregation in a blood sample is an issue that has been expected to be solved in an *in vitro* blood test.

At present, some solutions have been applied clinically to eliminate or reduce platelet pseudo-aggregation. For example, a blood sample with pseudo-aggregated platelets is heated to 37°C while shaking for a period of time, or additives are added to the blood sample to prevent platelet aggregation. These additives are, for example, anticoagulants (e.g., sodium citrate, heparin sodium, ACD, CTAD, CPT, or a mixture of CaCh and heparin sodium); platelet count diluents (a mixture containing such as sodium azide, calcium azide and sodium fluoride); anti-platelet drugs (added within 10 min after blood collection); and antibiotics of aminoglycoside (e.g., amikacin and kanamycin, which are added within 1 h after blood collection, but only effective for some samples).

These methods and agents for preventing platelet aggregation in an *in vitro* blood sample may lead to complicated test steps complicated, or may bring unsatisfactory platelet disaggregation effect, or are only effective for aggregation caused by certain reasons, and thus it is usually inevitably to re-collect blood and prepare a specific disaggregation agent. In practice, after the additives are added, a microscopic examination is further required to observe whether the platelets are disaggregated, so as to determine whether a disaggregation effect is sufficient for blood analysis. If necessary, the action time needs to be prolonged in an ice bath or a water bath to ensure a platelet disaggregation effect.

Therefore, there is still a need for a platelet disaggregation method and agent with simple operation and good universality during an *in vitro* blood test.

### SUMMARY

With regard to the above-mentioned problem of platelet pseudo-aggregation in an *in vitro* blood test, the inventors have surprisingly found that the addition of a solution that contains an amino group-containing compound to a blood sample may unexpectedly prevent platelet pseudo-aggregation and show a good disaggregation effect on a sample in which platelet pseudo-aggregation has occurred. Therefore, the disclosure is intended to provide the use of such substances for preventing and/or eliminating platelet aggregation in a blood sample in an *in vitro* blood test, and a platelet disaggregation agent for such use with simple and convenient operations, in which the platelet disaggregation agent can be applied to different modes of test and suitable for different apparatuses. The compound and agent for platelet disaggregation of the disclosure can eliminate multiple types of platelet aggregation, have stable and reliable effects, do not need repeated confirmation and have no negative influences on a normal blood test.

Therefore, in the first aspect, the disclosure provides use of a composition for preventing and/or eliminating

platelet aggregation in a blood sample in an *in vitro* blood test. The composition comprises at least one compound selected from the group consisting of a compound of formula (I) and a salt thereof:

R1-NH-R2 (I)

in which R1 and R2 are same or different and are each independently selected from the group consisting of H, $-SO_3H$, $-NH_2$, $-C(NH)-NH_2$, substituted or unsubstituted C1-16 alkyl, substituted or unsubstituted C6-10 aryl, substituted or unsubstituted C7-14 alkaryl, substituted or unsubstituted C7-14 aralkyl, -C(O)-Q1 and -C(O)-O-Q2, provided that R1 and R2 are not H simultaneously,

Q1 is H, $-NH_2$, substituted or unsubstituted C1-16 alkyl, substituted or unsubstituted C6-10 aryl, substituted or unsubstituted C7-14 alkaryl or substituted or unsubstituted C7-14 aralkyl, and

Q2 is H, substituted or unsubstituted C1-16 alkyl, substituted or unsubstituted C6-10 aryl, substituted or unsubstituted C7-14 alkaryl or substituted or unsubstituted C7-14 aralkyl,

in which the term "substituted" means being substituted with at least one selected from the group consisting of -NP1P2, $-SO_3H$, -OH, halogen, -CN, -C(O)-O-P3, -O-C1-16 alkyl, -O-C6-10 aryl, -O-C7-14 alkaryl, -O-C7-14 aralkyl, -C(O)-C1-16 alkyl, - C(O)-C6-10 aryl, -C(O)-C7-14 alkaryl, -C(O)-C7-14 aralkyl and -C(O)-NP1P2, in which -O-C1-16 alkyl, -O-C6-10 aryl, -O-C7-14 alkaryl, -O-C7-14 aralkyl, -C(O)-C1-16 alkyl, -C(O)-C6-10 aryl, -C(O)-C7-14 alkaryl and -C(O)-C7-14 aralkyl are unsubstituted or further substituted with at least one selected from the group consisting of $-NH_2$, -OH, $-SO_3H$, halogen, -CN, -COOH and $-C(O)NH_2$, respectively, and

P1, P2 and P3 are each independently selected from the group consisting of H, C1-16 alkyl, C6-10 aryl, C7-14 alkaryl and C7-14 aralkyl, in which C1-16 alkyl, C6-10 aryl, C7-14 alkaryl and C7-14 aralkyl are unsubstituted or further substituted with at least one selected from the group consisting of $-NH_2$, -OH, $-SO_3H$, halogen, -CN, - COOH and $-C(O)NH_2$, respectively.

According to an embodiment, in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-Q2, provided that R1 and R2 are not H simultaneously,

in which Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl, and

Q2 is H, substituted or unsubstituted C1-10 alkyl, substituted or unsubstituted C6-10 aryl, substituted or unsubstituted C7-10 alkaryl or substituted or unsubstituted C7-10 aralkyl,

in which the term "substituted" means being substituted with at least one selected from the group consisting of -NP1P2, $-SO_3H$, -OH, -CN, -C(O)-O-P3, -O-C1-10 alkyl, -O-C6-10 aryl, -O-C7-10 alkaryl, -O-C7-10 aralkyl and -C(O)-NP1P2, in which -O-C1-10 alkyl, -O-C6-10 aryl, -O-C7-10 alkaryl and -O-C7-10 aralkyl are unsubstituted or further substituted with at least one selected from the group consisting of $-NH_2$, -OH, $-SO_3H$, -CN, -COOH and $-C(O)NH_2$, respectively, and

P1, P2 and P3 are each independently selected from: H, C1-10 alkyl, C6-10 aryl, C7-10 alkaryl and C7-10 aralkyl, in which C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and $-C(O)NH_2$, respectively.

Preferably, in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-H, provided that R1 and R2 are not H simultaneously,

in which Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl,

in which the term "substituted" means being substituted with at least one selected from the group consisting of -NP1P2, $-SO_3H$, -OH, -CN, -C(O)-O-H and -C(O)-NP1P2, and

P1 and P2 are each independently selected from: H, C1-10 alkyl, C6-10 aryl, C7-10 alkaryl and C7-10 aralkyl, in which C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and $-C(O)NH_2$, respectively.

According to one embodiment, the compound of formula (I) has a total of 1-20 primary amino groups, secondary amino groups and/or imino groups.

The preventing and/or eliminating of the platelet aggregation in the blood sample is performed in a solution having a pH value of at least 3.0, preferably having a pH value of 7.5-11.

In the disclosure, the amino group of the compound of formula (I) has a pKa value of 1-16, preferably of 4-14.

According to a preferred embodiment, an amino group of the compound of formula (I) has a pKa value less than the pH value of the solution.

The compound of formula (I) has a concentration of 1-50 mmol/L, preferably 2-20 mmol/L in the solution.

According to another embodiment, the composition further comprises at least one ammonium ion-containing compound. Specifically, the ammonium ion-containing compound contains an anion selected from the group consisting of chloride ion, bromide ion, iodide ion, hydroxide, phosphate, hydrogen phosphate, dihydrogen phosphate, nitrate, Sulfhydryl, thiocyanate, sulfate, bisulfate, sulfite, bisulfite, carbonate, bicarbonate, formate, acetate, oxalate, propionate, malonate, citrate and a combination thereof. More specifically, the ammonium salt is at least one selected from ammonium chloride, ammonium bromide, ammonium iodide, ammonium phosphate, ammonium hydrogen phosphate, ammonium dihydrogen phosphate, ammonium nitrate, ammonium thiocyanate, ammonium bisulfite, ammonium oxalate, ammonium hydroxide, ammonium bisulfate and ammonium bicarbonate.

The ammonium ion-containing compound has a concentration of 1-50 mmol/L, preferably 2-20 mmol/L in preventing and/or eliminating the platelet aggregation in the blood sample.

According to an embodiment, the *in vitro* blood test comprises at least one of platelet detection, red blood cell detection and white blood cell detection. The red blood cell detection can further distinguish reticulocytes. The white blood cell detection further involves a detection of three categories (3-DIFF(differential)), four categories(4-DIFF(differential)) or five categories(5-DIFF(differential)).

More specifically, a red blood cell is at least one of a mature red blood cell, a reticulocyte and a nucleated red blood cell. A white blood cell is at least one of a neutrophil, an eosinophil, a basophil, a lymphocyte and a monocyte. An apparatus for the test may comprise an impedance detector and/or an optical detector.

The blood sample is taken from peripheral blood or venous blood of a mammal.

In the second aspect, the disclosure further provides the use of a composition for preventing and/or eliminating platelet aggregation in a blood sample in an *in vitro* blood test, in which the composition comprises at least one amino group-containing compound, and in a solution for preventing and/or eliminating the platelet aggregation in the blood sample, the amino group of the amino group-containing compound has a pKa value less than or equal to a pH value of the solution.

According to an embodiment, the amino group of the amino group-containing compound has the pKa value of 1-14.

In the disclosure, the amino group-containing compound has at least one of primary amino group and secondary amino group. Preferably, the amino group-containing compound has a total of 1-20 primary amino groups, secondary amino groups and/or imino groups.

According to the disclosure, the solution has the pH value of at least 3.0, preferably 7.5-11.

According to a specific embodiment, the amino group-containing compound is selected from the group consisting of a compound of formula (I) and a salt thereof:

R1-NH-R2 (I)

in which the compound of formula (I) is as defined above.

The amino group-containing compound has a concentration of 1-50 mmol/L, preferably 2-20 mmol/L in preventing and/or eliminating the platelet aggregation in the blood sample.

According to another embodiment, the composition further comprises at least one ammonium ion-containing compound. The ammonium ion-containing compound is as defined above.

The ammonium ion-containing compound may have a concentration of 1-50 mmol/L, preferably 2-20 mmol/L in the solution for preventing and/or eliminating the platelet aggregation in the blood sample.

Similarly, in the use, the *in vitro* blood test comprises at least one of platelet detection, red blood cell detection and white blood cell detection. The red blood cell detection further distinguishes reticulocytes. The white blood cell detection further includes a detection of three categories (3-DIFF(differential)), four categories(4-DIFF(differential)) or five categories(5-DIFF(differential)).

An apparatus for the test comprise an impedance detector and/or an optical detector.

The blood sample is taken from peripheral blood or venous blood of a mammal.

In the third aspect, the disclosure provides a agent for reducing platelet aggregation interference in an *in vitro* blood test, in which the agent comprises 1-50 mmol/L of at least one selected from the group consisting of a compound of formula (I) and a salt thereof, where the compound of formula (I) is as defined above.

Similarly, the agent also has a pH value of at least 3.0, preferably 7.5-11.

According to the disclosure, the amino group of the compound of formula (I) may have a pKa value of 1-16,

preferably 4-14. Preferably, the amino group of the compound of formula (I) has the pKa value less than or equal to the pH value of the solution.

Preferably, the compound of formula (I) has a concentration of 2-20 mmol/L.

According to yet another embodiment, the agent further comprises 1-50 mmol/L, preferably 2-20 mmol/L of at least one ammonium ion-containing compound. The ammonium ion-containing compound is as defined above.

Specifically, the agent further comprises a buffer, an osmotic pressure regulator and optionally at least one selected from a surfactant, a fluorescent dye and a red blood cell lytic agent.

In the fourth aspect, the disclosure further provides a method for preventing and/or eliminating platelet aggregation in a sample in an *in vitro* blood test, the method comprising treating the blood sample with the composition or agent as defined above. Optionally, the method further comprises testing the above-mentioned treated blood sample by using a hematology analyzer.

In the disclosure, the agent containing the compound, the above-mentioned use and the *in vitro* blood test method exhibit a disaggregation effect on aggregated platelet, and the platelet disaggregation takes effect within a short time without requiring provision of additional conditions such as temperature control with water bath, prolonged reaction time, or the like. Therefore, it is unnecessary to change the existing test operations and any hematology analyzer can be adopted for the test. Moreover, the agent has no adverse effects on the agents for platelet detection and other detections, exhibits a good disaggregation effect in multiple types of platelet aggregation circumstances, and can conveniently eliminate platelet pseudo-aggregation in the sample, and thus accurate blood cell detection parameters can be obtained.

## BRIEF DESCRIPTION OF THE DRAWINGS

FIG. 1 shows a schematic diagram of disaggregation and re-aggregation mechanisms of an amino group-containing compound on aggregated platelets;

FIG. 2 shows three-dimensional scatter diagrams of particles in a blood sample obtained by an optical detection apparatus, in which features of optical information of aggregated platelets and disaggregated platelets are shown respectively;

FIG. 3 shows a curve graph of disaggregation effects of amino group-containing compounds on aggregated platelets under different pH values;

FIG. 4 shows a curve graph of disaggregation effects of the amino group-containing compounds having different pKa values on aggregated platelets at a pH value of 9.5;

FIG. 5 shows a curve graph of induction effects of ADP at different final concentrations on platelet aggregation in a blood sample over time;

FIG. 6 shows a curve graph of disaggregation effects of an amino group-containing compound on platelet aggregation induced by ADP at different concentrations over time;

FIG. 7 shows a curve graph of induction effects of THR at different final concentrations on platelet aggregation in a blood sample over time;

FIG. 8 shows a curve graph of disaggregation effects of an amino group-containing compound on platelet aggregation induced by THR at different concentrations over time;

FIG. 9 shows a curve graph of induction effects of COL at different final concentrations on platelet aggregation in a blood sample over time;

FIG. 10 shows a curve graph of disaggregation effects of an amino group-containing compound on platelet aggregation induced by COL at different concentrations over time;

FIG. 11 shows a curve graph of induction effects of RIS at different final concentrations on platelet aggregation in a blood sample over time;

FIG. 12 shows a curve graph of disaggregation effects of an amino group-containing compound on platelet aggregation induced by RIS at different concentrations over time;

FIG. 13 shows disaggregation effects of various ammonium ion-containing compounds at different concentrations on aggregated platelets induced by ADP;

FIG. 14 shows disaggregation effects of 1-acetylguanidine, ammonium chloride and a combination thereof at different concentrations on aggregated platelets;

FIG. 15 shows three-dimensional scatter diagrams of platelets and red blood cells in a blood sample tested by using a conventional staining solution and a staining solution containing an amino group-containing compound in an optical detection apparatus before and after aggregation is induced in the sample;

FIG. 16 shows three-dimensional scatter diagrams of platelets and red blood cells in a blood sample tested by using a conventional diluent and a diluent containing an amino group-containing compound in an optical detection apparatus before and after aggregation is induced in the sample;

FIG. 17 shows three-dimensional scatter diagrams of platelets and red blood cells in a blood sample tested by using a conventional staining solution and a staining solution containing a combination of an amino group-containing compound and an ammonium ion-containing compound in an optical detection apparatus before and after aggregation is induced in the sample;

FIG. 18 shows three-dimensional scatter diagrams of platelets and red blood cells in a blood sample tested by using a conventional diluent and a diluent containing a combination of an amino group-containing compound and an ammonium ion-containing compound in an optical detection apparatus before and after aggregation is induced in the sample;

FIG. 19 shows three-dimensional scatter diagrams of white blood cells in a blood sample tested by using a conventional lytic solution and a lytic solution containing an amino group-containing compound in an optical detection apparatus before and after aggregation is induced in the sample;

FIG. 20 shows three-dimensional scatter diagrams of white blood cells in a blood sample tested by using a conventional lytic solution and a lytic solution containing a combination of an amino group-containing compound and an ammonium ion-containing compound in an optical detection apparatus before and after aggregation is induced in the sample; and

FIG. 21 shows a comparison diagram of disaggregation effects of a conventional diluent and a diluent containing an amino group-containing compound on a sample with platelet pseudo-aggregation in clinical practice.

## DESCRIPTION OF THE PREFERRED EMBODIMENTS

The technical solutions of the embodiments of the disclosure will be clearly and completely described below in conjunction with drawings and specific examples. Obviously, the embodiments described are merely some of the embodiments of the disclosure rather than all the embodiments. Based on the embodiments in the disclosure, all the other embodiments that would have been obtained by those of ordinary skill in the art without any inventive effort shall fall within the scope of protection of the disclosure.

Throughout the specification, unless otherwise specified, the terms used herein should be understood as the meanings commonly used in the art. Therefore, unless otherwise defined, all the technical and scientific terms used herein have the same meaning as commonly understood by those of skill in the art to which the disclosure belongs. In the event of a conflict, this specification takes precedence.

It should be noted that, in the disclosure, the term "comprise", "contain" or any other variant thereof is intended to encompass non-exclusive inclusion, so that a method or product including a series of elements not only includes the elements explicitly described, but also includes other elements not explicitly listed or elements inherent in the implementation of the method or product. In the absence of more restrictions, the element defined by the phase "comprising a ..." does not exclude the presence of a further related element in a method or device that includes the element.

Unless otherwise specified, the term "amino group" described herein refers to a primary amino group and/or a secondary amino group and does not include a tertiary amino group.

Unless otherwise specified, the term "amino group-containing compound" described herein refers to a compound containing at least one amino group (a primary amino group and/or a secondary amino group). The compound may further optionally contain a tertiary amino group and/or an imino group, and particularly an imino group.

The disclosure provides a class of amino group-containing compounds that can be used for platelet disaggregation. The inventors have surprisingly found that a class of amino group-containing compounds has significant disaggregation effects on platelet pseudo-aggregation caused by different reasons without additional heating or cooling, and without prolonged action time. Platelet aggregation can be eliminated after about 30 s by only adding a low amount of such substances to a solution of a blood sample to be tested. Therefore, the disclosure provides the use of such substances for preventing and/or eliminating platelet aggregation in a blood sample. Disaggregation of pseudo-aggregated platelets can not only obtain the real number of platelets in a blood sample, thereby providing a reliable reference basis for clinical decisions, but also eliminate the influence of large particles formed by aggregated platelets on the classification and count of other blood cells, particularly white blood cells.

Without wishing to be bound by the theory, the disaggregation principle of pseudo-aggregated platelets by the amino-containing compound of the disclosure is speculated to be that the amino group/imino group of the compound combines to the phosphatidylserine membrane of a platelet in a solution and bonds to carbonyl ester group (-O-C(O)-) of the phosphatidylserine membrane by hydrogen bond (see FIG. 1, which illustrates the disaggregation principle of the amino group-containing compound). In this way, the amino group of the compound bonding to the phosphatidylserine membrane blocks calcium ion channel and prevents the inflow of calcium ions. Furthermore, it is known that the platelet aggregation needs the inflow of calcium ions, and such compound prevents the inflow of calcium ions by the amino group to disaggregate the aggregated platelets. When the pH value is gradually decreased, the amino group of the compound is protonated. Although the specific reason is not clear, it is speculated that the protonated amino group on

the compound no longer bonds to a carbonyl ester group of the phosphatidylserine membrane by hydrogen bond, calcium ions can flow into the blood cell, and thus platelets are aggregated again. Theoretically, except for a tertiary amino group, a primary amino group, secondary amino group or imino group in which an N atom is connected to at least one hydrogen atom has a platelet disaggregation function to a certain extent.

Further research of the inventors has revealed that the disaggregation effect is improved with the number of amino groups/imino groups. In addition, a primary amino group, a secondary amino group and an imino group have sequentially decreased disaggregation effects. In general, the disaggregation effect of a highly hydrophilic compound is also higher than that of a weakly hydrophilic compound. A hydrophilic substituent such as hydroxyl group, carboxyl group, amido group or sulfonic group has no substantial influence on the disaggregation effect of an amino group/imino group, and may even, in some cases (e.g., hydroxyl and polyhydroxyl), be beneficial to the disaggregation. This may be related to the fact that these groups increase the solubility of the compound in water and the affinity of the compound to the phosphatidylserine membrane. A hydrocarbyl group such as an alkyl group and an aryl group has little influence on platelet disaggregation as long as it does not influence the solubility of the compound in water to a certain extent. A hydrophobic group such as ester group (-C(O)-O-) and carbonyl group (-C(O)-) reduces the disaggregation effect to some extent.

Therefore, the compounds having a group such as hydroxyl, carboxyl or sulfonic group, such as substances of alcohol amines, amino acids, sulfonic acids or the like, are preferable. In addition, the compounds having a plurality of amino groups and/or imino groups, such as substances of guanidines, short peptides or the like, are also preferable. Preferably, the amino group-containing compound has at least one of a primary amino and a secondary amino, preferably 1-20 of a sum of primary amino groups, secondary amino groups and imino groups. Preferably, the amino group-containing compound has at least one primary amino, preferably 1-4 primary amino groups.

According to the mechanism as speculated above, when the amino group-containing compound is used for disaggregation, the amino group represents in a deprotonated form in the solution. The amino /imino groups in different compounds have different combination abilities with hydrogen ions in solution. Such abilities to combine with/dissociate from hydrogen ions can be represented by a dissociation constant pKa.

The amino group-containing compound has the following equilibrium formula in a solution:

$$R1R2NH_2^+ \leftrightarrow R1R2NH + H^+$$

$$pKa = lg\ ([R1R2NH] \times [H^+]/[R1R2NH_2^+]).$$

In order to obtain a suitable platelet disaggregation effect, the amino group of the compound has an appropriate pKa. For the compound having a plurality of amino groups/imino groups, the pKa of amino group described herein refers to the first dissociation constant, that is, pKa of amino group or imino group which firstly dissociates hydrogen ion.

Generally, the amino group of the compound has a pKa value of 1-16, preferably 1-14, more preferably 4-14.

In addition, with respect to a same compound, when pKa of amino group of the compound is less than pH of the solution, the disaggregation ability is improved. According to the above-mentioned principle of disaggregation of aggregated platelets by the compound, it can be understood that when pH is gradually increased, the balance of combination and dissociation of an amino group of the compound with hydrogen ion in the solution moves toward the dissociation. Therefore, more amino groups are deprotonated, as such more amino groups combine with the phosphatidylserine membrane of platelet via hydrogen bonds, thereby blocking the inflow of calcium ions, and thus the compound plays the role in disaggregation. Moreover, such binding further promotes the deprotonation of more amino groups of the compounds, so that the disaggregation can be completed within a short time without elevating the temperature rise.

In general, during an *in vitro* blood test, pH of a test sample can be changed in a large range according to the requirement. However, in order to obtain an excellent disaggregation effect, in the use of the disclosure, preventing and/or eliminating platelet pseudo-aggregation is performed in a solution with pH of at least 3.0, preferably at least 7.0, more preferably 7.5-11, particularly preferably 9.5-11. The pH value of the solution can be adjusted according to the sepcies of the compound and the requirements of the test so as to obtain a desired disaggregation effect.

The effective concentration of the amino group-containing compound that is sufficient to disaggregate pseudo-aggregated platelets is related to the number and type of the amino group(s) of the compound, and also related to factors such as the hydrophilicity of the compound. In general, the concentration of the amino group-containing compound for platelet disaggregation is in a range of 1-50 mmol/L, such as 1 mmol/L, 2 mmol/L, 5 mmol/L, 10 mmol/L, 15 mmol/L, 20 mmol/L, 25 mmol/L, 30 mmol/L, 35 mmol/L, 40 mmol/L and 50 mmol/L. According to a preferred embodiment, the concentration is in a range of 2-20 mmol/L, more preferably in a range of 5-15 mmol/L.

The disclosure provides the use of the amino group-containing compound, particularly the compound represented by structural formula (I) and a salt thereof, for preventing and/or eliminating platelet pseudo-aggregation in a blood sample in an *in vitro* blood test. Provided is a compound of formula (I).

R1-NH-R2 (I)

R1 and R2 are same or different and are each independently selected from the group consisting of H, $-SO_3H$, $-NH_2$, $-C(NH)-NH_2$, substituted or unsubstituted C1-16 alkyl, substituted or unsubstituted C6-10 aryl, substituted or unsubstituted C7-14 alkaryl, substituted or unsubstituted C7-14 aralkyl, -C(O)-Q1 and -C(O)-O-Q2, provided that R1 and R2 are not H simultaneously.

Q1 is H, $-NH_2$, substituted or unsubstituted C1-16 alkyl, substituted or unsubstituted C6-10 aryl, substituted or unsubstituted C7-14 alkaryl or substituted or unsubstituted C7-14 aralkyl.

Q2 is H, substituted or unsubstituted C1-16 alkyl, substituted or unsubstituted C6-10 aryl, substituted or unsubstituted C7-14 alkaryl or substituted or unsubstituted C7-14 aralkyl.

The expression "substituted" means being substituted with at least one selected from the group consisting of -NP1P2, $-SO_3H$, -OH, halogen, -CN, -C(O)-O-P3, -O-C1-16 alkyl, -O-C6-10 aryl, -O-C7-14 alkaryl, -O-C7-14 aralkyl, -C(O)-C1-16 alkyl, - C(O)-C6-10 aryl, -C(O)-C7-14 alkaryl, -C(O)-C7-14 aralkyl and -C(O)-NP1P2, in which -O-C1-16 alkyl, -O-C6-10 aryl, -O-C7-14 alkaryl, -O-C7-14 aralkyl, -C(O)-C1-16 alkyl, -C(O)-C6-10 aryl, -C(O)-C7-14 alkaryl and -C(O)-C7-14 aralkyl are unsubstituted or further substituted with at least one selected from the group consisting of $-NH_2$, -OH, $-SO_3H$, halogen, -CN, -COOH and $-C(O)NH_2$, respectively.

P1, P2 and P3 are each independently selected from the group consisting of H, C1-16 alkyl, C6-10 aryl, C7-14 alkaryl and C7-14 aralkyl, in which C1-16 alkyl, C6-10 aryl, C7-14 alkaryl and C7-14 aralkyl are unsubstituted or further substituted with at least one selected from the group consisting of $-NH_2$, -OH, $-SO_3H$, halogen, -CN, - COOH and $-C(O)NH_2$, respectively.

According to one embodiment, in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-Q2, provided that R1 and R2 are not H simultaneously, in which Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl, and Q2 is H, substituted or unsubstituted C1-10 alkyl, substituted or unsubstituted C6-10 aryl, substituted or unsubstituted C7-10 alkaryl or substituted or unsubstituted C7-10 aralkyl,

The expression "substituted" means being substituted with at least one selected from the group consisting of -NP1P2, $-SO_3H$, -OH, -CN, -C(O)-O-P3, -O-C1-10 alkyl, -O-C6-10 aryl, -O-C7-10 alkaryl, -O-C7-10 aralkyl and -C(O)-NP1P2, in which -O-C1-10 alkyl, -O-C6-10 aryl, -O-C7-10 alkaryl and -O-C7-10 aralkyl are unsubstituted or further substituted with at least one selected from the group consisting of $-NH_2$, -OH, $-SO_3H$, -CN, -COOH and $-C(O)NH_2$, respectively, and P1, P2 and P3 are each independently selected from: H, C1-10 alkyl, C6-10 aryl, C7-10 alkaryl and C7-10 aralkyl, in which C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and $-C(O)NH_2$, respectively.

Preferably, in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-H, provided that R1 and R2 are not H simultaneously, in which Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl.

The expression "substituted" means being substituted with at least one selected from the group consisting of -NP1P2, $-SO_3H$, -OH, -CN, -C(O)-O-H and -C(O)-NP1P2, and P1 and P2 are each independently selected from: H, C1-10 alkyl, C6-10 aryl, C7-10 alkaryl and C7-10 aralkyl, in which C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and $-C(O)NH_2$, respectively.

Further, in the compound of formula (I), R1 and R2 are same or different and are each independently selected from the group consisting of H, $-C(NH)-NH_2$, substituted or unsubstituted C1-6 alkyl, substituted or unsubstituted phenyl, substituted or unsubstituted benzyl, substituted or unsubstituted phenethyl and -C(O)-Q1, provided that R1 and R2 are not H simultaneously, where Q1 is as defined above.

Still further, in the compound of formula (I), Q1 is substituted or unsubstituted C1-6 alkyl, substituted or unsubstituted phenyl, substituted or unsubstituted benzyl or substituted or unsubstituted phenethyl, in which the expression "substituted" means being substituted with at least one selected from the group consisting of -NP1P2, $-SO_3H$, -OH and -C(O)-NP1P2, and P1, P2 and P3 are each independently selected from: H, C1-6 alkyl, phenyl, benzyl and phenethyl, in which C1-6 alkyl, phenyl, benzyl and phenethyl are unsubstituted or further substituted with at least one selected from the group consisting of $-NH_2$, -OH, $-SO_3H$, -COOH and $-C(O)NH_2$.

C1-16 alkyl described herein may be a linear or branched alkyl, preferably C1-14 alkyl or C1-12 alkyl, further preferably C1-10 alkyl, more preferably C1-6 alkyl. Examples of the alkyl include, but are not limited to, methyl, ethyl, n-propyl, isopropyl, n-butyl, isobutyl, tert-butyl, n-pentyl, iso-pentyl, neopentyl, n-hexyl, n-octyl, dodecyl and hexadecyl.

C6-10 aryl described herein may be phenyl or naphthyl.

C7-14 alkaryl described herein may be monoalkyl- or polyalkyl-substituted aryl, preferably C7-10 alkaryl, such as, but not limited to, methylphenyl, ethylphenyl, propylphenyl, butylphenyl, dimethylphenyl, methylethylphenyl and diethylphenyl.

C7-14 aralkyl described herein may be phenylalkyl, preferably C7-10 aralkyl, such as, but not limited to, benzyl, phenylethyl, phenylpropyl, and phenylbutyl.

The expression "substituted" described herein means being substituted with at least one of the substituents defined above. With regard to amino substituent, -NP1P2, multiple amino substituents may be preferable according to the disclosure. The substituents may involve up to 20 amino groups, for example, 1-18 amino groups, such as 2, 3, 4, 5, 6, 8, 10, 12, 14 or 16 amino groups. Preferably, the amino group is a primary amino group and/or a secondary amino group. More preferably, it is a primary amino group. Hydroxyl substituent, -OH, is a preferable substituent. The number of hydroxyl substitutions may be 1-6, such as 2, 3, 4, 5 or 6. In addition, sulfonic group, carboxyl group and nitrile group are also preferable.

The halogen described herein generally refers to -F, -Cl, -Br or -I, preferably -F, - Cl or -Br.

Preferred examples of the amino group-containing compound of the disclosure include guanidines, alcohol amines (particularly polyhydroxyl alcohol amines, such as 1,3-diamino-2-propanol), amino acids (preferably lysine, glutamine and glycine), oligopeptides (e.g., a dipeptide) or polyamino-substituted highly branched or linear alkanes (e.g., 2-(aminomethyl)-propane-1,3-diamine, 1,3-diaminopropane, and the like).

Examples of the compound of formula (I) for preventing and/or eliminating platelet pseudo-aggregation include, but are not limited to, sulfonic acids, e.g., sulfamic acid, aminomethylsulfonic acid, taurine, sulfanilic acid and 2,5-diaminobenzenesulfonic acid; amino acids, e.g., glutamic acid, glutamine, arginine, lysine, alanine, glycine, N-tris(hydroxymethyl)methylglycine and 4-aminobutyric acid; oligopeptides, e.g., a dipeptide, such as lys-lys and gly-gly; alcohol amines/phenolamines (particularly polyhydroxy-substituted amines), e.g., ethanolamine, triethanolamine, 3-amino-1-propanol, 4-amino-1-butanol, 4-hydroxybenzylamine, tyramine (4-hydroxyphenethylamine), 1,3-diamino-2-propanol, 3-amino-1,2-propanediol, tris(hydroxymethyl)aminomethane, p-hydroxyaniline, 3,5-dihydroxyaniline, 4-aminophenol; arylamines, e.g., m-phenylenediamine and m-phenylenetriamine; alkylamines, e.g., ethylamine, n-propylamine, n-butylamine, tert-butylamine, 1,3-diaminopropane, 2-(aminomethyl)-propane-1,3-diamine; aminonitriles, e.g., aminoacetonitrile and triaminopropionitrile; amides, e.g., formamide, acetamide, carbamide, asparagine methyl ester, 2-(methylamino)succinamide, 2-aminoacetamide, 2-aminopropanamide and glutamine; and guanidines, e.g., guanidine, biguanide, aminoguanidine, methylguanidine, {[amino(imino)methyl]amine}-acetic acid and 1-acetylguanidine, but not limited thereto.

Preferably, the compound of formula (I) is a hydroxyl-substituted amine, an alkylamine, a benzenesulfonamide, an amino acid, a short peptide or a guanidine.

A salt of the amino group-containing compound, particularly a salt of the compound containing an acidic group such as sulfonic group, carboxyl group or the like, also fall within the scope of the disclosure. The salt may be a salt of an alkali metal (e.g., potassium or sodium), a salt of an alkaline earth metal (e.g., magnesium), a salt of an ammonium cation, etc., and may also be an inner salt. As will be detailed below, the salt of ammonium cation is particularly preferable.

The disclosure provides use of a composition for preventing and/or eliminating platelet pseudo-aggregation in a blood sample during an *in vitro* blood test. The composition comprises at least one amino group-containing compound as mentioned above, particularly the compound of formula (I) or a salt thereof, and of course, can also comprise two or more amino group-containing compounds.

The composition comprising at least one amino group-containing compound as mentioned herein may be in a form of the compound itself or in a form of a solution. For example, the compound may be dissolved in an appropriate solvent so as to be conveniently applied to the use of the disclosure. The appropriate solvent may be water, alcohol (e.g., ethanol), an aqueous solution of alcohol or other commonly used solvents that do not interfere with the test of the blood sample.

The at least one amino group-containing compound plays a role in platelet disaggregation at a certain concentration in the solution containing a blood sample. In the use of the disclosure, as mentioned above, the effective concentration of the amino group-containing compound for platelet disaggregation is in a range of about 1-50 mmol/L, preferably 2-20 mmol/L, more preferably 5-15 mmol/L. If two or more compounds are used, the total concentration of the compounds falls within the above range. As mentioned above, there are many factors causing the platelet pseudo-aggregation, and the degree of aggregation and difficulty of disaggregation are also very different. Therefore, the amino group-containing compound, particularly the compound of formula (I) described above, may be used within a large concentration range. A compound having a strong disaggregation ability (e.g., a compound having relatively more amino groups and/or showing a good affinity to the cell membrane) may be used at a relatively low concentration in a situation where disaggregation is relatively easy. On the contrary, a compound having weak disaggregation ability may be used at a relatively high concentration in a situation where disaggregation is difficult or the degree of aggregation is high. The selection of a suitable concentration can be reasonably determined by those skilled in the art by reference to the following

specific examples in conjunction with actual needs.

In addition, according to the further finding of the inventors, when the amino group-containing compound is used in combination with an ammonium ion-containing compound, a better platelet disaggregation effect can be achieved.

Therefore, the composition further includes at least one ammonium ion-containing compound. The ammonium ion-containing compound contains an anion selected from the group consisting of chloride ion, bromide ion, iodide ion, hydroxide, phosphate, hydrogen phosphate, dihydrogen phosphate, nitrate, hydrosulfide, thiocyanate, sulfate, bisulfate, sulfite, bisulfite, oxalate, sulfite, bisulfite, carbonate, bicarbonate, formate, acetate, oxalate, propionate, malonate, citrate and a combination thereof.

Examples of the ammonium ion-containing compound may be, but not limited to, ammonium chloride, ammonium bromide, ammonium iodide, ammonium phosphate, ammonium hydrogen phosphate, ammonium dihydrogen phosphate, ammonium nitrate, ammonium thiocyanate, ammonium bisulfite, ammonium oxalate, ammonium hydroxide, ammonium bisulfate or ammonium bicarbonate.

The composition may comprise one or more of the ammonium ion-containing compounds. Preferably, the ammonium ion-containing compound is, e.g., ammonium chloride, ammonium bromide, ammonium phosphate, and ammonium hydrogen phosphate.

When the composition further comprises the ammonium ion-containing compound, the ammonium ion-containing compound may have a concentration in a range of 1-50 mmol/L, such as 1 mmol/L, 2 mmol/L, 5 mmol/L, 10 mmol/L, 15 mmol/L, 20 mmol/L, 25 mmol/L, 30 mmol/L, 35 mmol/L, 40 mmol/L or 50 mmol/L. Preferably, the ammonium ion-containing compound has a concentration in a range of 2-20 mmol/L, more preferably in a range of 5-15 mmol/L.

The inventors found that the ammonium ion also has a good platelet disaggregation effect under the above-mentioned pH condition. Preferably, when the composition comprises the ammonium ion-containing compound, the pH value is preferably at least 7.0, more preferably 7.5-11, particularly preferably 9.5-11.0. With respect to the combination of the ammonium ion-containing compound with the amino group-containing compound, low concentrations thereof can achieve a good disaggregation effect. That is, when the total concentration of the ammonium ion-containing compound and the amino group-containing compound is equal to the concentration of either of the compounds used alone, the combination of the two compounds may achieve a better effect. In other words, the combination of the two compounds has a synergistic effect on platelet disaggregation. Therefore, when the two compounds are used in combination, the total concentration may be in a range of 2-50 mmol/L, such as 1 mmol/L, 2 mmol/L, 5 mmol/L, 10 mmol/L, 15 mmol/L, 20 mmol/L, 25 mmol/L, 30 mmol/L, 35 mmol/L, 40 mmol/L and 50 mmol/L. Preferably, the composition may comprise 1-20 mmol/L of the amino group-containing compound and 1-20 mmol/L of the ammonium ion-containing compound. More preferably, the composition may comprise 1-10 mmol/L of the amino group-containing compound and 1-10 mmol/L of the ammonium ion-containing compound, and even may comprise 1-5 mmol/L of the amino group-containing compound and 1-5 mmol/L of the ammonium ion-containing compound. There is no particular limitation on the ratio of the two compounds. As an example, the molar ratio of the amino group-containing compound to the ammonium ion-containing compound may be in a range of 1:10 to 10:1, preferably in a range of 1:4 to 4:1, such as 1:4, 1:2, 1:1, 2:1 and 4:1. Most preferably, the molar ratio of the two is 1:1.

Without wishing to be bound by theory, the platelet disaggregation mechanism of ammonium ions is still unclear. It is speculated that this mechanism is related to the concentration of hydrated ammonia formed by ammonium ions in the solution. However, although ammonia water also has fairly good disaggregation performance, its disaggregation performance is worse than that of the ammonium ion-containing compound, particularly an ammonium salt such as ammonium chloride, ammonium phosphate, ammonium halide or the like. Therefore, it is speculated that some types of anions in the solution may also play the role in promoting platelet disaggregation. This is different from the speculated disaggregation mechanism of the amino group-containing compound.

As can be seen from the following examples, when the amino group-containing compound and the ammonium ion-containing compound are used in combination, the disaggregation effect is significantly improved.

This combination is particularly beneficial for an *in vitro* blood test because the concentration of the substance for in platelet disaggregation is significantly reduced. It is well known that the surfactant of a quaternary ammonium salt can damage cell membranes, particularly cell membranes of red blood cells, and are therefore commonly used as red blood cell lytic agent. The inventors observed that the composition has no damage effect on red blood cells within the above-mentioned concentration range, and the red blood cell count is generally unchanged no matter the composition is used or not used. Particularly, at preferable concentrations of the amino group-containing compound and the ammonium ion-containing compound used in combination, the composition not only can obtain an excellent platelet disaggregation effect, but also has no adverse effects on blood cells, particularly has no adverse effects on red blood cells.

According to the use of the disclosure, the *in vitro* blood test refers to a test on blood cells such as platelets, red blood cells and white blood cells in a blood sample. In the use of the disclosure, the test may be performed by means of a microscopic examination or by an apparatus, preferably by an apparatus. Specifically, the test may involve, for example, detecting various cells in the blood by using a hematology analyzer and obtaining corresponding detection information. In the use of the disclosure, there is no limitation on the hematology analyzer, and any apparatus for an *in*

*vitro* blood test can be applied to the use of the disclosure.

In the use of the disclosure, there is no limitation on a detector; for example, an impedance detector and an optical detector can be used.

By taking the optical detector as an example, multiple aggregated platelets is recognized as one particle in an optical channel of the hematology analyzer. Therefore, the particle of such aggregated platelets has a larger volume and a stronger fluorescence intensity than a single platelet, and thus the number of platelets with particles of aggregated platelets is smaller than that of non-aggregated platelets. As reflected in detection information, referring to the three-dimensional scatter diagrams obtained in a Mindray hematology analyzer as shown in FIG. 2, it can be seen that a sample having aggregated platelets shows relatively large forward-scattered (FS) light intensity information of the platelts, reflecting that the particle volume is relatively large. In addition, the fluorescence intensity (FL) is relatively high. When the volume of some of the particles of aggregated platelets is equal to the volume of white blood cells, the white blood cell count is also influenced. After disaggregation, the volume of platelets recognized by particle signals in the optical channel is decreased, the fluorescence intensity is decreased, and the number is increased. Further referring to FIG. 2, as can be seen from the three-dimensional scatter diagrams, it is reflected from the particle swarm of platelets after disaggregation that the platelet volume corresponding to the forward-scattered light (FL) is obviously decreased, the number of particles is increased, and the fluorescence intensity is decreased.

With regard to the impedance detector, similarly, multiple aggregated platelets are regarded as one particle to generate one electrical signal. Since the volume of the particle becomes larger after aggregation, the particle cannot be recognized as a platelet, thereby resulting in a low platelet count. In addition, aggregated platelets also can influence the white blood cell count, the red blood cell count and alter the histogram. This influence is very obvious in an early three categories hematology analyzer.

According to the use of the disclosure, by adding the above-mentioned composition to the sample, platelet aggregation can be prevented, and the aggregated platelets can be disaggregated, so that the accurate platelet count can be obtained.

In addition, since aggregated platelets no longer exist, the interference with the detection of white blood cells can also be eliminated simultaneously, so that accurate white blood cell detection information can be obtained. The white blood cell detection further comprises classifying and/or counting of, e.g., nucleated cells, basophils, eosinophils, neutrophils and/or lymphocytes.

The "detection information" described herein includes raw signals (e.g., electrical pulse, light intensity), processed information such as histograms, scatter diagrams or the like, and finally-reported categories and/or counts information.

In addition, since the number of red blood cells in the blood is much larger than that of other species of cells, platelet aggregation has little influence on red blood cells in optical detections and thus has almost no influence on the count result. However, the addition of a substance of an amine and an ammonium salt may lead to lytic of red blood cells. In embodiments of the disclosure, it can be seen from the following examples that the amino group-containing compound and further the ammonium ion-containing compound within the ranges of the concentrations defined above do not have negative influences on the sample environment (e.g., pH and ionic strength), and also do not cause damage to blood cells. Therefore, after the platelet disaggregation, the red blood cell detection will not be influenced. According to the use of the disclosure, mature red blood cells, reticulocytes and nucleated red blood cells can be further classified and counted, respectively.

The use of the disclosure has an effect on platelet aggregation caused by different reasons, and there is no particular limitation on the sample applied to the use. The sample may be taken from peripheral blood, such as peripheral blood or venous blood. There is also no particular limitation on the way to obtain the sample. For example, the blood can be collected by pricking fingertip or can be collected into an anticoagulation tube from the vein. Fresh blood can be collected into an anticoagulation tube coated with a conventional anticoagulant such as EDTA, because the composition has a good effect on platelet pseudo-aggregation induced by EDTA.

In general, the collected blood sample can be subjected to any conventional treatment to obtain a test sample. The treatment may be, e.g., dilution treatment, staining treatment and lytic treatment. In the use of the disclosure, the composition can be added to the sample separately as a treatment agent (e.g., as a platelet disaggregation agent), or can be added to the sample together with the corresponding treatment agent in any step of treating the sample. Therefore, the composition may form an individual treatment agent. The amino group-containing compound and optionally the ammonium ion-containing compound can also be added to an agent for dilution, staining or lytic treatment to form a diluent agent, staining agent or lytic agent having the platelet disaggregation effect, thereby simultaneously achieving the effect of preventing/eliminating platelet disaggregation in any step of treatment without influence of the treatment. The disaggregation reaction of platelets by the composition does not require additional heat preservation (e.g., low temperature or heating). Platelet disaggregation can be completed in about 30 s after the treatment agent containing the composition is evenly mixed with the sample, and then the sample can be tested.

According to the disclosure, the sample may be derived from mammal, preferably primate, more preferably

human.

Therefore, the disclosure further provides a method for preventing and/or eliminating platelet aggregation in a sample in an *in vitro* blood test. The method comprises a treatment of preventing/eliminating platelet aggregation interference in the blood sample. In the treatment, the blood sample is treated with a composition or an agent comprising the above-mentioned amino group-containing compound and optionally the ammonium ion-containing compound under the above-mentioned conditions.

More specifically, the disclosure provides an *in vitro* blood test method against platelet aggregation interference.

The method may comprise treating a blood sample with a disaggregation agent comprising at least one selected from the amino group-containing compound and optionally the ammonium ion-containing compound to prevent and/or eliminate platelet aggregation in the blood sample, and testing the blood sample treated by the disaggregation agent with a hematology analyzer so as to at least obtain the detection information of blood cells after eliminating the interference with the blood cells by the aggregation.

The blood cells may be platelets, red blood cells and/or white blood cells.

The detection information may be, depending on the detector, pulse signals or optical signals of particles in the sample. The hematology analyzer may process the detection information to further obtain detection parameters such as histograms, scatter diagrams, categories and/or counts of blood cells.

According to one embodiment, the hematology analyzer may include an impedance detector. The method may comprise the following operations: subjecting the blood sample treated with the disaggregation agent to an impedance detector in the hematology analyzer so as to obtain electrical signals of particles in the blood sample and further obtain detection parameters of platelets and/or red blood cells.

In addition, the test still adopts the impedance detector, and the blood sample is further lysed with a red blood cell lytic agent in addition to being treated with the disaggregation agent before the test is performed, and the method comprises: subjecting the blood sample treated with the disaggregation agent and the red blood cell lytic agent to the impedance detector in the hematology analyzer so as to obtain electrical signals of particles in the blood sample and further obtain detection parameters of white blood cells.

Three categories and count of white blood cells can be obtained by the impedance detector.

According to another embodiment, the hematology analyzer includes an optical detector, and the method comprises subjecting the blood sample treated with the disaggregation agent to an optical detector in the blood analyzer so as to obtain at least one scattered light intensity signal of particles in the blood sample and further obtain detection parameters of platelets. Preferably, the at least one scattered light intensity signal is a forward-scattered light intensity signal. More preferably, the detection parameters of platelets can be obtained by using forward- and side-scattered light intensity signals.

According to yet another embodiment, the optical detector is still adopted, the blood sample is further stained with a fluorescent dye before the test is performed, and the method comprises: subjecting the blood sample treated with the disaggregation agent and the fluorescent dye to the optical detector in the hematology analyzer, and obtaining a fluorescence intensity signal and at least one scattered light intensity signal of particles in the blood sample so as to obtain detection parameters of platelets and/or red blood cells.

Preferably, in this embodiment, the method further comprises distinguishing reticulocytes and obtaining detection parameters of reticulocytes. More preferably, the at least one scattered light intensity signal is a forward-scattered light intensity signal. The detection parameters of platelets and/or red blood cells can also be obtained by using the fluorescence intensity signal, the forward-scattered light intensity signal and the side-scattered light intensity signal.

According to a further embodiment, the optical detector is still adopted, the blood sample is further stained by a fluorescent dye and lysed by a red blood cell lytic agent, and the method comprises: subjecting the blood sample treated with the disaggregation agent, the fluorescent dye and the red blood cell lytic agent to the optical detector in the hematology analyzer, and obtaining at least two optical signals of particles in the blood sample so as to obtain the detection parameters of white blood cells.

Preferably, in this embodiment, the at least two optical signals are selected from the fluorescence intensity signal, the forward-scattered light intensity signal and the side-scattered light intensity signal.

In the detection of white blood cells, four categories (i.e., eosinophils, neutrophils, monocytes and lymphocytes) can be obtained by using a DIFF detection channel of for example, Mindray hematology analyzer, or the detection information of basophils is further obtained by using a WNB channel of the Mindray hematology analyzer.

In the above-mentioned test methods, the disaggregation agent, the fluorescent dye and the red blood cell lytic agent may be an individual agent or may be arbitrarily combined as a mixed agent. For example, the disaggregation agent may form an agent having the platelet disaggregation effect, such as a diluent, a staining agent, a lytic agent, a staining-lytic agent, etc.

Therefore, the disclosure further provides an agent for reducing platelet aggregation interference in an *in vitro* blood test.

Since the volume of the agent for treating the blood sample is much larger than the volume of the blood sample

itself during the *in vitro* blood test, the concentration, pH and other treatment conditions in the above-mentioned use are generally also applicable to the agent for reducing platelet aggregation interference in the disclosure.

According to the disclosure, the agent may comprise 1-50 mmol/L of at least one amino group-containing compound, and may further comprise 1-50 mmol/L of at least one ammonium cation-containing compound. The concentration defined in the disclosure, with respect to two or more amino group-containing compounds, is the total concentration thereof. Similarly, the concentration is the total concentration for two or more ammonium ion-containing compounds. The at least one amino group-containing compound is as defined above and is therefore not reiterated here, and its concentration is preferably 2-20 mmol/L, more preferably 5-10 mmol/L.

The at least one ammonium ion-containing compound which is further contained is also as defined above, and its concentration is preferably 2-20 mmol/L, more preferably 5-10 mmol/L. According to a preferred embodiment, when the agent contains both the amino group-containing compound and the ammonium ion-containing compound, the concentrations of the two compounds can independently vary in a range of 1-20 mmol/L, 1-10 mmol/L, or even 1-5 mmol/L. The molar ratio of the amino group-containing compound to the ammonium ion-containing compound may be any ratio in a range of, for example, 1 : 10 to 10 : 1, preferably 1 : 4 to 4 : 1.

There is no particular limitation on pH of the agent, as it is related to use requirements. Preferably, the agent has a pH value of at least 3.0, at least 7.0, preferably 7.5-11, particularly preferably 9.5-11. The pH of the agent can be adjusted by a conventional buffer. The disclosure has no particular limitation on the type of a buffer used for the agent. For example, a citric acid buffer pair, a phosphate buffer pair, a boric acid buffer pair, a phthalate buffer pair, a 3-(N-morpholine)ethanesulfonic acid buffer pair and a 4-hydroxyethylpiperazine ethanesulfonic acid buffer pair can be used.

The agent shall have a certain osmotic pressure. The osmotic pressure may vary according to actual requirements of the agent. For example, for a diluent agent, the osmotic pressure may be in a range of 180-240 mOsm/L, preferably 200 mOsm/L. For a lytic agent, the osmotic pressure may be in a range of 70-130 mOsm/L, preferably 90 mOsm/L. The agent may comprise an osmotic pressure regulator. The agent of the disclosure has no particular limitation on osmotic pressure regulators. For example, the osmotic pressure regulator may be, but not limited to, inorganic salts such as sodium chloride and potassium chloride, sugars such as glucose, mannose, fructose and maltose, or the like.

The agent may further comprise a surfactant. The agent may comprise a surfactant with a function according to different test purposes. For example, for detection of red blood cells, the agent as a diluent may comprise a surfactant allowing the spheroidization of red blood cells. Such surfactant is, but not limited to, for example, N-alkyl betaine, sodium N,N-dimethyloctadecylamine hydrochloride and sodium dodecylbenzenesulfonate. For detection of white blood cells, the agent may comprise a surfactant that alters the permeability of cell membranes, thereby facilitating a nucleic acid dye to pass through the cell membranes and combine with nucleic acids in the cells. Such surfactant is, but not limited to, for example, phenoxyethanol, sodium dioctadecylamine hydrochloride and sodium N-methylamide carboxylate.

In addition, the agent may further comprise other necessary components such as a preservative, an antibacterial agent, a cell membrane protective agent and a chelating agent according to requirements. Such components may be selectively added by those skilled in the art according to the need.

According to an embodiment of the disclosure, the agent may be used as a diluent for the blood sample. For example, the agent may be a diluent for impedance detection or also may be a diluent for optical detection. A formulation of such diluent may include, for example,

| | |
|---|---|
| Amino group-containing compound | 1-50 mmol/L |
| Ammonium ion-containing compound | 0 or 1-50 mmol/L |
| Buffer | 0.1-1 g/L |
| Osmotic pressure regulator | 1-10 g/L |
| Surfactant | 1-10 g/L. |

According to the disclosure, the agent may be a staining agent. Generally, a dye is stored separately in a form of an organic solution in an apparatus for platelet detection as the dye is easily dissolved in an organic solvent. If necessary, the dye may also be added to the diluent and made into a staining agent. The dye in the staining agent may be a nucleic acid dye. Examples of the nucleic acid dye may be, but not limited to, an asymmetric cyanine dye, thiazole orange TO, oxazole orange YO, acridine orange AO, etc., especially, such as PI, DAPI, Hoechst series (e.g., Hoechst33258 and Hoechst33342) or the like. Preferably, the nucleic acid dye is an asymmetric cyanine dye, such as SYBR Green. The pH value that achieves optimum efffect of such dye is substantially consistent with the pH value that achieves an optimum effect of the amino group-containing compound and the ammonium ion-containing compound in the disclosure. According to different detection requirements, the dye may also be a mitochondria dye (e.g., Janus Green B, MitoLite Red, Rhodamine 123, Mitotracker Green, Mitotracker Deep Red, Mitotracker Red and the like) or a membrane bye (e.g., DiA, DiD, DiI, DiO, DiR, DiS, FDA, Alexa Fluor 488, Super Fluor 488 and the like).

A formulation of such staining agent may include:

| | |
|---|---|
| Amino group-containing compound | 1-50 mmol/L |
| Ammonium ion-containing compound | 0 or 1-50 mmol/L |
| Buffer | 0.1-1 g/L |
| Osmotic pressure regulator | 1-10 g/L |
| Surfactant | 1-10 g/L |
| Nucleic acid dye | 0.01-10 g/L. |

In addition, the agent of the disclosure may be a lytic agent for detection of white blood cells. The lytic agent used as the agent may be any conventional red blood cell lytic agent. As mentioned above, the amino group-containing compound in the agent of the disclosure and especially the ammonium ion-containing compound, have no adverse effects on red blood cells in the defined concentration ranges, as such the species, concentration, use mode of the lytic agent used in the agent are the same as those in the prior art. Examples of the red blood cell lytic agent comprise, but not limited to, quaternary ammonium salts (e.g., hydrocarbonylbenzyldimethylammonium chloride, dodecyltrimethylammonium chloride and alkylbenzyldimethylammonium chloride). The formula of such lytic agent may include:

| | |
|---|---|
| Amino group-containing compound | 1-50 mmol/L |
| Ammonium ion-containing compound | 0 or 1-50 mmol/L |
| Buffer | 0.1-1 g/L |
| Osmotic pressure regulator | 1-10 g/L |
| Surfactant | 1-10 g/L |
| Red blood cell lytic agent | 0.1-10 g/L |

Again, according to the above, when the agent that comprises an amino group-containing compound and may further comprise an ammonium ion-containing compound of the disclosure is used for preventing/eliminating platelet pseudo-aggregation, the concentration of active ingredients is low, no additional reaction condition (e.g., cooling or heating) is required, the reaction time does not need to be prolonged, and platelet disaggregation can be completed within 30 s. Therefore, the compounds have no adverse effects on the blood test, have no influence on the test operations and conditions, and can eliminate the interference of platelet aggregation by using the existing apparatuses and test methods.

**Example 1. Comparison of abilities for disaggregate platelets of amino group-containing compounds and amino group-free compounds with similar structures**

This example compared platelet disaggregation effects of amino group-containing compounds and amino group-free compounds with similar structures and substituents. The tested compounds were shown in Table 1 below.

Table 1: Tested amino group-containing compounds and amino group-free compounds with similar structure

| Amino group-containing compound | Amino group-free compound | Amino group-containing compound | Amino group-free compound |
|---|---|---|---|
| sulfanilic acid | benzenesulfonic acid | 4-aminophenol | phenol |
| 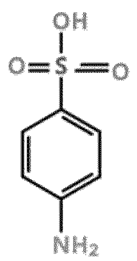 | 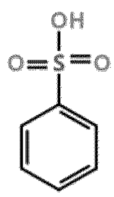 | 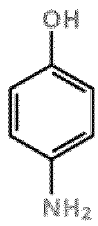 | 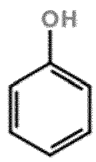 |
| ethanolamine | ethanol | glycine | acetate |

(continued)

| Amino group-containing compound | Amino group-free compound | Amino group-containing compound | Amino group-free compound |
|---|---|---|---|
| 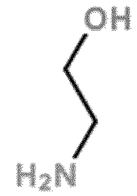 | 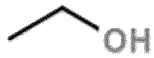 | 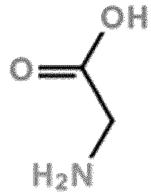 | 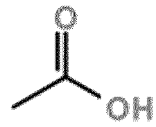 |
| acetamide | acetaldehyde | guanidine | formaldehyde |
| 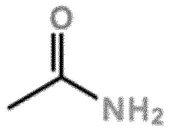 | 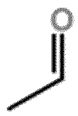 | 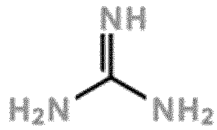 | 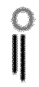 |
| methylsulfanilic acid | ethanesulfonic acid | sarcosine | butyric acid |
| 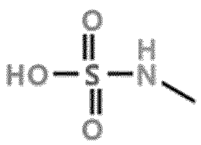 | 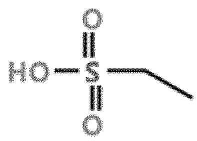 | 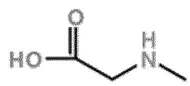 | 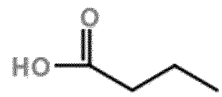 |
| 2-(methylamino) ethanol | butanol | 4-[(methylamino) methyl] phenol | 4-butylphenol |
| 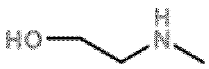 | 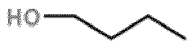 | 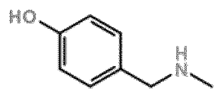 | 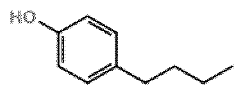 |
| diacetamide | acetylacetone | | |
| 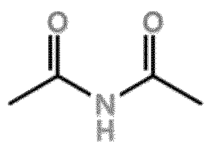 | 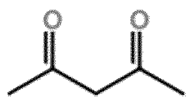 | | |

The compounds in Table 1 above were respectively added to a diluent used for a hematology analyzer to prepare blood sample diluents. Platelet-aggregated blood samples were obtained by treating blood samples with a platelet aggregation inducer ADP at a final concentration of 0.01 mmol/L. The composition of the diluent was as follows: citric acid (0.5 g/L), surfactant phenoxyethanol (0.1 g/L), bacteriostatic agent (6 g/L), sodium chloride (3 g/L) and EDTA (0.1 g/L). Each of the compounds in Table 1 (at a final concentration 0.01 mol/L) was added to the diluent separately, pH was adjusted to 14, and the osmotic pressure was adjusted to 200 mOsm/L to obtain the treatment solution.

The venous blood of a healthy subject was collected into blood anticoagulant tubes for later use. Two aliquot blood samples (1 ml/sample) were taken. The above-mentioned diluent was added to one of the samples, the resulting solution was mixed evenly, and then the platelet count was detected in a hematology analyzer (Mindray BC-6000Plus) and recorded as: PLT_O (unaggregated). The platelet aggregation inducer ADP (at a final concentration of 0.01 mmol/L) was added to the other sample, and the resulting solution was mixed evenly. Platelets were aggregated 5 min later, at this time the above-mentioned diluent was added, and then the blood containing the aggregated platelets was detected by the hematology analyzer to obtain the platelet count, which was recorded as: PLT_O (aggregated). Another 1 ml of blood sample was taken, the platelet aggregation inducer ADP (at a final concentration of 0.01 mmol/L) was added, and the resulting solution was mixed evenly. The above-mentioned treatment solution was added 5 min later, the resulting solution was mixed evenly, and then the platelet count of the treated blood was detected by the hematology analyzer and was recorded as: PLT_O (disaggregated).

In general, PLT_O (undisaggregated) < PLT_O (disaggregated) < PLT_O (unaggregated).

The term "disaggregation rate" mentioned herein was calculated by the following equation:

with respect to disaggregated samples:

$$\text{disaggregation rate} = \text{PLT_O (disaggregated)/PLT_O (unaggregated)};$$

or

the control disaggregation effect of the above-mentioned diluent on the sample in which platelet aggregation was induced can be calculated by the following equation:

with respect to undisaggregated (control) sample:

$$\text{disaggregation rate} = \text{PLT_O (undisaggregated)/PLT_O (unaggregated)}.$$

The platelet disaggregation rates were determined one by one according to the above-mentioned method after treating the ADP-induced blood with a treatment solution containing a compound in Table 1, and the results were shown in Table 2 below.

Table 2. Platelet disaggregation effects of amino group-containing compounds and amino group-free compounds with similar structures

| Amino group-containing compound | | Amino group-free compound | |
|---|---|---|---|
| name | Platelet disaggregation rate | Name | Platelet disaggregation rate |
| sulfanilic acid | 55.15% | benzenesulfonic acid | 13.77% |
| 4-aminophenol | 67.73 | phenol | 8.44% |
| ethanolamine | 91.45% | ethanol | 14.46% |
| glycine | 65.47% | acetate | 11.75% |
| acetamide | 64.57% | acetaldehyde | 16.54% |
| guanidine | 84.35% | formaldehyde | 13.85% |
| methylsulfanilic acid | 53.49% | ethanesulfonic acid | 16.54% |
| sarcosine | 64.75% | butyric acid | 11.45% |
| 2-(methylamino) ethanol | 82.49%% | butanol | 13.14% |
| 4-[(methylamino) methyl] phenol | 59.43% | 4-butylphenol | 9.43% |
| diacetamide | 75.43% | acetylacetone | 8.45% |
| Blank control: | | NaCl | 10.45% |

As can be seen from Table 2 above, only the amino group-containing compounds had the disaggregation effect on the aggregated platelets, whereas other groups such as a sulfo group, a hydroxyl group, a carboxyl group and a carbonyl group had almost no disaggregation effect on the aggregated platelets (comparable to the disaggregation rate of the blank control NaCl). It can be determined from this example that in each compound, the amino group played a major role in platelet disaggregation.

**Example 2. Relationship between number of amino groups and ability to disaggregate platelets**

This example compared influences on platelet disaggregation of different numbers of amino groups in compounds having similar structures. The compounds containing different numbers of amino groups were tested respectively according to the method of Example 1, and the platelet disaggregation rate and pKa value of each compound were shown in Table 3.

Table 3. Platelet disaggregation rates and pKa values of compounds containing different numbers of amino groups

| Compound name | Structural formula | Number of amino groups | pKa | Platelet disaggregation rate |
|---|---|---|---|---|
| sulfanilic acid | 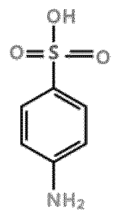 | 1 | 3.32 | 55.15% |
| 2,5-diaminobenzenesulfonic acid | 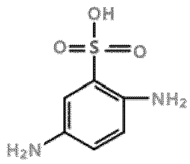 | 2 | 5.17 | 62.44 |
| glycine | 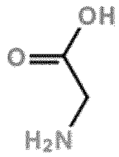 | 1 | 9.77 | 65.47% |
| lysine | 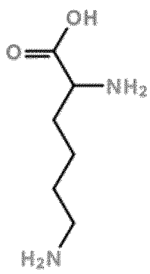 | 2 | 10.75 | 70.49% |
| lys-lys | 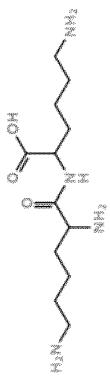 | 4 | 11.01 | 85.75% |
| ethanolamine | 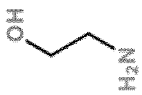 | 1 | 9.44 | 86.45% |
| 1,3-diamino-2-propanol | 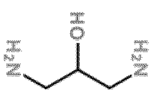 | 2 | 9.68 | 93.78% |

(continued)

| Compound name | Structural formula | Number of amino groups | pKa | Platelet disaggregation rate |
|---|---|---|---|---|
| aniline | 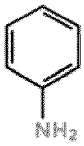 | 1 | 4.58 | 56.26% |
| m-phenylenediamine | 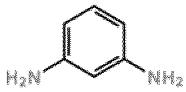 | 2 | 4.67 | 66.83% |
| 1,3,5-triaminobenzene | 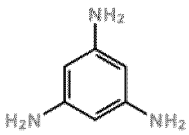 | 3 | 5.50 | 72.44% |
| butylamine | 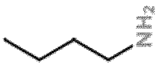 | 1 | 10.59 | 87.53% |
| 1,3-diaminopropane | 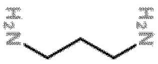 | 2 | 10.4 | 91.14% |
| 2-(aminomethyl)-propane-1,3-diamine | 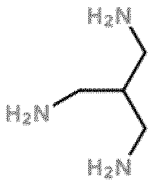 | 3 | 9.59 | 96.43% |
| acetamide | 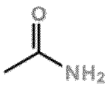 | 1 | 15.1 | 44.44% |
| asparagine methyl ester | 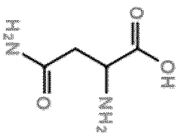 | 2 | 9.13 | 61.43% |
| 2-(methylamino) succinamide | 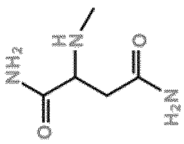 | 3 | 7.02 | 74.43% |
| guanidine | 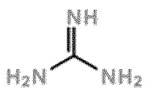 | 3 | 13.17 | 84.35% |
| aminoguanidine | 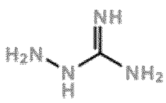 | 4 | 11.04 | 92.35% |
| biguanide | 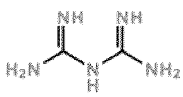 | 5 | 13.25 | 97.54% |

As can be seen from Table 3 above, the disaggregation effects on the aggregated platelets of the compounds, which have similar or same structures and were only different in numbers of substituted amino groups, were increased with the increased number of amino groups, where oligopeptides, polyamino-substituted alkanes, polyamino-substituted hydroxylamines and guanidines were more preferable.

**Example 3. Relationship between pka of amino group and ability to disaggregate platelets at the same pH environment**

This example compared influences on platelet disaggregation of different pKa values of amino groups in compounds having similar structures. The tests were performed substantially according to the method of Example 1, except that pH was adjusted to a suitable one with respect to different compounds. The compounds having different numbers of amino groups were tested respectively, and the platelet disaggregation rate and pKa value of each compound were shown in Table 4.

Table 4. Platelet disaggregation rates of compounds containing amino groups with different pKa values at certain ambient pHs

| Compound name | Structural formula | pH | pKa | Platelet disaggregation rate |
|---|---|---|---|---|
| sulfamic acid | 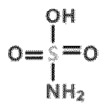 | 5.75 | 1.19 | 64.54% |
| aminomethanesulfonic acid | 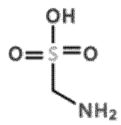 | 5.75 | 5.75 | 52.44% |
| taurine | 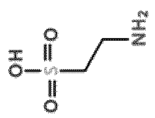 | 5.75 | 9.12 | 46.25% |
| glycine | 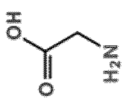 | 10.33 | 9.77 | 64.35% |
| alanine | 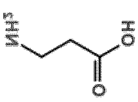 | 10.33 | 10.33 | 49.65% |
| 4-aminobutyric acid | 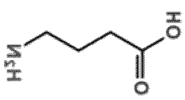 | 10.33 | 10.43 | 46.46% |
| ethanolamine | 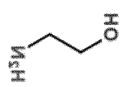 | 9.96 | 9.44 | 88.45% |
| 3 -amino-1-propanol | 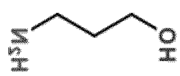 | 9.96 | 9.96 | 81.43% |
| 4-amino-1-butanol | 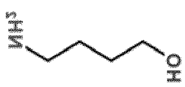 | 9.96 | 10.35 | 74.45% |
| 4-aminophenol | 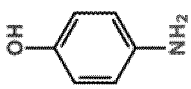 | 9.14 | 5.5 | 67.37% |

(continued)

| Compound name | Structural formula | pH | pKa | Platelet disaggregation rate |
|---|---|---|---|---|
| 4-hydroxybenzylamine | 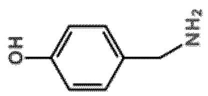 | 9.14 | 9.14 | 51.43% |
| tyramine | 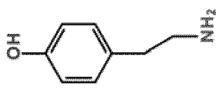 | 9.14 | 10.62 | 48.44% |
| ethylamine | 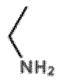 | 10.64 | 10.67 | 83.32 |
| butylamine | 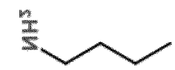 | 10.64 | 10.64 | 84.34% |
| n-propylamine | 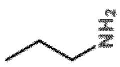 | 10.64 | 10.58 | 85.53% |
| 2-aminoacetamide | 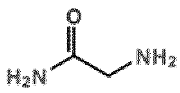 | 8.18 | 7.93 | 64.57% |
| 2-aminopropanamide | 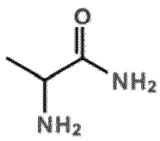 | 8.18 | 8.18 | 62.65% |
| glutamine | 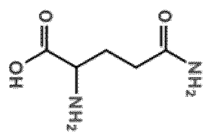 | 8.18 | 9.13 | 59.25% |
| methyl guanidine | 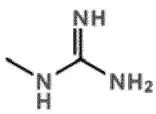 | 10.8 | 13.2 | 86.36% |
| {[amino(imino)methyl] amino}<br>-acetic acid | 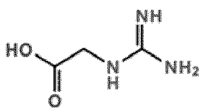 | 10.8 | 10.8 | 94.02% |
| 1-acetylguanidine | 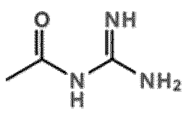 | 10.8 | 8.33 | 97.35% |

As can be seen from Table 4 above, different substituents on amino groups influenced the pKa values of the amino groups.

**Example 4. Relationship between ambient pH and pKa of amino groups in amino group-containing compounds, and influences thereof on disaggregation ability**

As can be seen from example 3, there was a relationship between the ambient pH and the pKa of amino groups of an amino group-containing compound. In order to further study the relationship between the ambient pH and the pKa of amino groups of the amino group-containing compound and the influences thereof on the ability to disaggregate

platelets, in this example 1-acetylguanidine having a pKa of 8.33 is selected and tested according to the method similar to that in Example 1, except that the ambient pH value varied in a range of 7.0-10. The curve graph of the pH vs. the disaggregation rate was obtained as shown in FIG. 3.

As can be seen from FIG. 3, the ambient pH is strongly related to the pka of the substance for the platelet disaggregation. When pH increased, the disaggregation ability of the amino group-containing substance was enhanced and the disaggregation rate was increased from 35.23% to 97.32%. When pH reached above 9.5, the disaggregation rate generally remained unchanged. As can be seen, when the ambient pH is greater than the pKa of amino groups of the compound to a certain extent, increasing pH continuously had little influence on the disaggregation effect. This is consistent with the previously speculated disaggregation mechanism. That is, a majority of amino groups of the compound exists in a deprotonated form in the solution at a suitable pH, thereby promoting or even accelerating the disaggregation.

Further, with the similar method, the disaggregation rates of aggregated platelets treated with the compounds with different pKa values of amino group were tested at pH 9.5, and the results were as shown in FIG. 4. Some compounds having pKa values between 8 and 11 were selected: N-tris(hydroxymethyl)methylglycine (8.1), arginine (9.0), glutamic acid (9.6), glycine (9.6), sulfanilic acid (10.1) and lysine (10.7). When the pKa of amino group was increased from 8.1 to 10.7, the disaggregation effect of each compound was gradually reduced.

As can be seen from this example, adjusting the pH value to be greater than the pKa value of amino groups helps to improve the disaggregation effect. Therefore, the ability of the amino group-containing compound to disaggregate the aggregated platelets can be improved by adjusting the ambient pH value. On the other hand, a suitable amino group-containing compound can be selected according to the need of the test environment.

**Example 5. Disaggregation effects of structures of amino group-containing compounds on aggregated platelets**

In order to investigate the influences of compound structures on the disaggregation of aggregated platelets, compounds having representative structures were selected and tested at pH = 9.5 according to the method of Example 1, and the disaggregation rate was obtained for each compound, as shown in Table 5 below.

Table 5. Disaggregation effects of different compounds on aggregated platelets

| Name | Structures/ number | Amino type/ number | pKa | Disaggregati on rate |
|---|---|---|---|---|
| gly-gly | carboxyl/1<br>carboxyl/1 | primary amino/1<br>secondary amino/1 | 9.6 | 57.29% |
| glutamic acid | carboxyl/2 | primary amino/1 | 9.6 | 63.24% |
| glycine | carboxyl/1 | primary amino/ 1 | 9.6 | 70.47% |
| 2-(aminomethyl)-propane-1,3-diamine | --- | primary amino/3 | 9.6 | 97.46% |
| arginine | carboxyl/1 | primary amino/2<br>secondary amino/1<br>imino/1 | 9.0 | 62.64% |
| 1-acetylguanidine | carbonyl/1 | primary amino/1<br>secondary amino/1<br>imino/1 | 8.3 | 97.11% |
| tris(hydroxymethyl)amino methane | hydroxyl/3 | primary amino/1 | 8.1 | 97.32% |
| tris(hydroxymethyl)amino methane acetate | carboxyl/1<br>hydroxyl/3 | secondary amino/1 | 8.1 | 75.23% |
| trihydroxymethyl(N,N-dihydroxymethylamino) methane | hydroxyl/5 | tertiary amino/1 | 6.5 | 24.0% |

As can be obviously seen from Table 5 above, the interaction between amino group and cell membrane was influenced by the structure of the amino group-containing substance. For example, gly-gly had the same pKa to glycine and even had one more secondary amino group, but the disaggregation effect of gly-gly was still slightly lower than that of glycine. This can be attributed to the presence of one more carbonyl group in the molecule. In addition, both glutamic acid and glycine had only one primary amino group with a pKa of 9.6, but glutamic acid had one more carboxyl group in comparison with glycine and also had a relatively low disaggregation rate.

As can be seen from Table 5, carbonyl and carboxyl do not seem to be helpful to the improvement of platelet disaggregation effects of the compounds, and alkyl chains had little influence on the disaggregation effects.

In addition, the disaggregation effect is improved with the number of amino groups/imino groups, particularly primary amino groups. Arginine has more amino groups/imino groups, but the disaggregation effect thereof is not very good, which may be related to the weakened function of amino groups caused by its ability to form a cyclic lactam.

Moreover, hydroxyl seems to be more conducive to enhancing the disaggregation effects of amino groups in the compounds. For example, tris(hydroxymethyl)aminomethane acetate only contains one secondary amino group, but the disaggregation rate thereof is still higher than that of glycine. Tris(hydroxymethyl)aminomethane having a primary amino group has an even better disaggregation effect on platelets. It was speculated that hydroxyl facilitates the approach of compounds to the cell membranes of platelets, and the platelet disaggregation effect of primary amino group is better than that of secondary amino groups. Of course, the high disaggregation rate is also related to the low pKa (8.1) of these two compounds.

In addition, the tertiary amino group has almost no disaggregation effect. From the above mechanism, it can be understood that if there is no hydrogen atom on an amino group can form a hydrogen bond, the amino group will be difficult to play its role.

**Example 6. Disaggregation effects of amino group-containing compounds on different platelet aggregation models**

Platelet aggregation can be induced by a variety of substances, thereby leading to blood coagulation. In order to investigate the disaggregation effects of amino group-containing compounds on platelet aggregation induced by different factors, in this example, substances, which can induce platelet aggregation, in the blood coagulation test scheme were selected according to the existing platelet aggregation pathways, and a variety of platelet aggregation models were established. The substances included adenosine diphosphate (ADP), thrombin (THR), collagen (COL) and ristocetin (RIS). It was found from this example that ADP, THR, COL and RIS respectively induced different degrees of platelet aggregation with the changes of concentrations and time. In addition, the amino group-containing compounds can also effectively disaggregate the platelets having different inducements and aggregation degrees in certain concentration ranges and time ranges.

FIG. 5 shows the relationship between the degrees of platelet aggregation induced by ADP at different concentrations (0.01-1 mM) and the time. Specifically, several samples of 1 ml of venous blood were taken, and ADP was added to each sample to make the final concentrations be 0.01 mM, 0.25 mM, 0.5 mM and 1 mM respectively. The blood containing aggregated platelets was tested by using a hematology analyzer. The features including numbers, sizes, etc., of various cell particles in the blood were obtained by means of an impedance channel and an optical channel in the hematology analyzer. Normal blood was collected into blood anticoagulant tubes, and then the number of platelet particles detected by the impedance channel in the hematology analyzer was obtained and recorded as (PLT_I). When platelet aggregation occurred, the volume of a particle of the aggregated platelets was much larger than the volume of a single platelet and therefore the accurate count cannot be achieved by means of a corresponding platelet recognition mechanism. As a result, the platelet count was lowered and recorded as PLT_I (aggregated). No amino group-containing compound was added to the diluent of the impedance channel, thus the impedance channel reflected the degree of platelet aggregation in this aggregation model. The platelet count before aggregation was recorded as PLT_I (unaggregated), the platelet count after aggregation was recorded as PLT_I (aggregated), and the aggregation rate = PLT_I (aggregated)/PLT_I (unaggregated). The number of platelet particles tested by means of the optical channel in the hematology analyzer was obtained and recorded as (PLT_O). When platelet aggregation occurred, the volume of a particle of the aggregated platelets was much larger than the volume of a single platelet and therefore the accurate count cannot be achieved by means of a corresponding platelet recognition mechanism. As a result, the PLT_O (unaggregated) was lowered. An amino group-containing compound was added to the diluent of the optical channel to achieve the disaggregation of aggregated platelets, and the PLT_O (disaggregated) after disaggregation was obtained. The platelet count before aggregation was recorded as PLT_O (unaggregated), and the platelet count after aggregation was recorded as PLT_O (undisaggregated). Similarly, PLT_ O (undisaggregated) < PLT_ O (disaggregated) < PLT_ O (unaggregated), and the disaggregation rate = PLT_ O (disaggregated)/PLT_ O (unaggregated). On the basis of the above principle, the model was established, and the disaggregation effect of the amino group-containing compound was quantitatively determined.

As can be seen from FIG. 5, platelet aggregation generally reached the maximum degree within 5 min after ADP was added. The degree of platelet aggregation was increased with the amount of ADP added. The influences of ADP at a final concentration of 0.25 mM or above on the degree of platelet aggregation were roughly the same. In addition, ADP at a concentration of 0.01 mM had the weakest influence on the degree of platelet aggregation and can achieve at most about 35% of platelet aggregation, but platelet disaggregation was relatively slow at this concentration.

Further referring to FIG. 6, it shows curves of the platelets disaggregation effects of the amino group-containing

compound in blood samples induced by ADP at different concentrations over time. Specifically, according to the method similar to Example 1, the amino group-containing compound (1-acetylguanidine) was added to a diluent to treat blood samples in which different concentrations of aggregation inducer were added. Then the samples were tested, in which the test was performed every 5 min after the treatment solution was added to each sample so as to obtain the change of disaggregation rates over time. The diluent was composed of citric acid (0.5 g/L), surfactant phenoxyethanol (0.1 g/L), a bacteriostatic agent (6 g/L), sodium chloride (3 g/L), EDTA (0.1 g/L) and 1-acetylguanidine (10 mmol/L), and had pH of 9.5 and osmotic pressure of 200 mOsm/L.

As can be seen from FIG. 6, the same compound at the same concentration had remarkable disaggregation effects on platelet aggregation with various aggregation degrees. The disaggregation effects became the highest immediately after the amino group-containing compound was added, and was decreased over time, but generally remained stable after 10 min. In addition, the lower concentration of ADP, the more stable the effect. Considering that the hematology analyzer can quickly complete the test of the sample with only a small amount of sample, the test may be started immediately within 5 min, preferably within 2 min, more preferably in 30 s after the treatment solution containing the amino group-containing compound was added.

Therefore, the degree of aggregation can be increased by increasing the concentration of ADP, thereby increasing the difficulty of disaggregation.

Similarly, FIG. 7 and FIG. 8 show curve graphs of platelet aggregation tests induced by THR at different concentrations. The substantially same method as the above-mentioned was used, except that ADP was replaced by THR. The concentrations of THR were 0.1 U, 0.15 U, 0.2 U, 0.23 U, 0.25 U, 0.28 U, 0.30 U and 0.35 U, respectively. As can be seen, the platelet aggregation effect was weak when the concentration of THR was low, and the platelet aggregation rate increased rapidly after 1 min when the concentration of THR was high. This was because after a certain concentration of THR was added, soluble fibrinogen converted to insoluble fibrinogen, causing blood coagulation, and platelets were also wrapped by insoluble fibrinogen, resulting in a sharp increase in aggregation rate. Meanwhile, the disaggregation effect of 1-acetylguanidine reached the highest immediately each concentration of THR, and decreased significantly after 1 min, which was also related to the inability of the disaggregation agent to play its role at this time. However, a good disaggregation rate can be achieved when samples were tested immediately after treatment.

FIG. 9 and FIG. 10 show curve graphs of platelet aggregation tests induced by COL at different concentrations. The substantially same method as the above-mentioned was used, except that ADP was replaced by COL. The concentrations of COL were 0.05 mM, 0.1 mM, 0.5 mM, 1 mM, 5 mM, 10 mM and 20 mM, respectively. As can be seen, COL at various concentrations can quickly make the degree of platelet aggregation close to 100%, where the lower the concentration was, the faster the aggregation was. In addition, the disaggregation effects of 1-acetylguanidine on samples treated with COL at different concentrations (except for the sample treated with COL at the lowest concentration) reached the highest immediately, and decreased significantly after about 4-8 min. This was also because the soluble fibrinogen converted to insoluble fibrinogen after COL was added, thereby causing blood coagulation.

FIG. 11 and FIG. 12 show curve graphs of platelet aggregation tests induced by RIS at different concentrations. The substantially same method as the above-mentioned was used, except that ADP was replaced by RIS. The concentrations of RIS were 0.15 mM, 0.375 mM, 0.75 mM, 1.5 mM and 3 mM, respectively. As can be seen, the induction effects of RIS on platelet aggregation were exerted after a long period of time, and the degree of platelet aggregation reached the highest after 15-20 min. In addition, the disaggregation effect of 1-acetylguanidine can reach the highest immediately for RIS at various concentrations, and remained relatively stable within 10 min. The rapid decrease in the disaggregation rate after 15 min was also because the blood in the samples was coagulated.

As can be seen from each platelet aggregation induction model above, the aggregation inducers had different influences on the degree of platelet aggregation via different pathways, but the amino group-containing compound can act immediately on the aggregation caused by any of the inducers. However, since the conversion of the soluble fibrinogen to insoluble fibrinogen caused blood coagulation, the disaggregation effects on the samples in which platelet aggregation induced by THR or COL could not exhibited after the blood coagulated. However, this had little influence on classification and count of platelets determined by a hematology analyzer.

This example shows that the amino group-containing compound has good disaggregation effects at certain concentration and time ranges in multiple types of platelet aggregation circumstances and can all take effects rapidly, and the test can be immediately and easily performed without any additional reaction condition (e.g., heating or longer reaction time).

**Example 7. Disaggregation effects of ammonium cation-containing compounds on aggregated platelets**

This example determined the disaggregation effects of ammonium cation-containing compounds on aggregated platelets.

The test was performed according to the same method as in Example 1, except that the compounds in Table 1 were replaced by ammonium ion-containing compounds (i.e., ammonium chloride, ammonium bromide, ammonium

iodide, ammonium phosphate, ammonium hydrogen phosphate, ammonium dihydrogen phosphate, ammonium thiocyanate, ammonium bicarbonate, ammonium oxalate and ammonia water) and the control compound was NaCl. The pH of the treatment solution was adjusted to 9.5 and the osmotic pressure was adjusted to 200 mOsm/L. The disaggregation rates of ammonium ion-containing compounds at different concentrations were determined as shown in FIG. 13.

As can be seen from FIG. 13, in the samples with platelet aggregation induced by 0.01 mmol/L ADP, each ammonium ion-containing compound produced a certain disaggregation effect when the concentration reached 1 mmol/L, and achieved the best effect when the concentration reached 10 mmol/L. Specifically, at a concentration of 10 mmol/L, the disaggregation effect of $(NH_4)_2HPO_4$ was 99.25%, the disaggregation effect of $NH_4CL$ was 95.70%, the disaggregation effect of $(NH_4)_3PO_4$ was 94.87%, the disaggregation effect of $NH_4Br$ was 93.48%, the disaggregation effect of $NH_4I$ was 92.75%, the disaggregation effect of $NH_4HCO_3$ was 88.68%, the disaggregation effect of $NH_4C_2O_4$ was 87.67%, the disaggregation effect of $NH_4H_2PO_4$ was 86.24%, the disaggregation effect of $NH_4SCN$ was 85.78% and the disaggregation effect of $NH_4OH$ was 83.05%. When the ammonium ion-containing compound was replaced by NaCl, the disaggregation effect dropped to 18.90%. As can be seen, ammonium ions rather than anions play the role in platelet disaggregation. Moreover, different anions have some influence on the disaggregation, but the influence is not significant.

### Example 8. Influence of ambient pH values on ability to disaggregate platelets by ammonium ion-containing compounds

This example studied the influences of ambient pH values on the disaggregation effect on aggregated platelets by an ammonium ion-containing compound. According to a method similar to that in Example 1, the compounds in Table 1 were replaced by 0.01 mol/L ammonium chloride and the pH values of the treatment solutions were adjusted to 4.0, 5.5, 6.5, 7.5, 8.5, 9.5 and 11.0, the disaggregation rates of platelets in the samples treated with ammonium chloride were tested, as shown in Table 6 below.

Table 6: Influences of pH values on platelet disaggregation effects of ammonium ions in ammonium chloride

| pH value | 4.0 | 5.5 | 6.5 | 7.5 | 8.5 | 9.5 | 11.0 |
|---|---|---|---|---|---|---|---|
| disaggregation rate | 18.62% | 31.70% | 59.61% | 94.05% | 94.62% | 95.70% | 97.72% |

As seen in Table 6, the disaggregation effects of ammonium ions on aggregated platelets were suddenly increased to 94.05% when the ambient pH value was 7.5 or above, and the disaggregation rates were remained at high levels when the pH value was in a range of 7.5-11.0. This suggested that platelet disaggregation can be promoted in the case that more of ammonium ions in the solution form hydrated ammonia.

### Example 9. Platelet Disaggregation effects of a combination of amino group-containing compound and ammonium ion-containing compound

On the basis that the amino group-containing compound and the ammonium ion-containing compound may disaggregate the aggregated platelets with different disaggregation mechanisms, this example determined the platelets disaggregation effect of a combination of an amino group-containing compound and an ammonium ion-containing compound. According to a method similar to that in Example 1 except that the compounds in Table 1 were replaced by ammonium chloride, 1-acetylguanidine, the combination of ammonium chloride and 1-acetylguanidine, and NaCl as a control in concentrations of 1 mmol/L, 2 mmol/L, 5 mmol/L, 10 mmol/L, 20 mmol/L and 50 mmol/L respectively, in which in the combination of ammonium chloride and 1-acetylguanidine, the concentrations of ammonium chloride and 1-acetylguanidine were equal and the total concentration thereof meets the concentration requirement above. In addition, in order to highlight the disaggregation effect of the combination of the two compounds, 0.1 mmol/L ADP (that is, 10 times the concentration of ADP in Example 1) was used to induce platelet aggregation in this example, and the ability of the amino group-containing compound and the ammonium ion-containing compound to disaggregate platelets was studied under an increased difficulty of disaggregation. The disaggregation rates were determined as shown in FIG. 14.

The results showed (FIG. 14) that the disaggregation effects of $NH_4Cl$ and 1-acetylguanidine on platelet aggregation induced by ADP at a final concentration of 0.1 mmol/L were gradually enhanced with the increase of concentrations, respectively. For example, the disaggregation effect of 50 mmol/L of $NH_4Cl$ used alone was 66.27%, the disaggregation effect of 50 mmol/L 1-acetylguanidine used alone was 62.14%, and the disaggregation effect of $NH_4Cl$ and 1-acetylguanidine used in combination at a total concentration of 50 mmol/L reached 95.48%. The combination of the two compounds had a much enhanced platelet disaggregation effect. This enhanced effect was also seen at other concentrations of the combination. This suggests that the two compounds have a synergistic effect on platelet disaggregation. Therefore, when the two compounds are used in combination, they are preferably used at lower concentrations

(e.g., as low as 1-20 mM, as low as 1-10 mM, or even as low as 1-5 mM, respectively).

Experiments on the use of the agent containing amino group-containing compounds of the disclosure for reducing platelet disaggregation interference were further performed in actual tests below.

**Test Example 1. Amino group-containing compound in staining solution for detection of platelets and red blood cells**

This test example provided a staining solution for a hematology analyzer, and the staining solution can be used to eliminate platelet aggregation interference in detection of red blood cells and platelets. This test example and the following test examples all used the hematology analyzer (BC-6000Plus) from Shenzhen Mindray Bio-medical Electronics Co., Ltd. Such staining solution had the formulations below.

Control staining solution:

| | |
|---|---|
| SYBR Green | 50 mg/L, |
| citric acid | 0.5 g/L, |
| surfactant phenoxyethanol | 0.1 g/L, |
| bacteriostatic agent 1,3-dimethylurea | 6.0 g/L, |
| sodium chloride | 3.0 g/L. |

Staining solution B:

1-acetylguanidine was added in the control staining solution with the final concentration of 0.01 mol/L.

The control staining solution and staining solution B were prepared with distilled water at 25°C, with pH adjusted to 9 and the osmotic pressure of 200 mOsm/L.

1 ml of human venous blood sample collected into an anticoagulant tube was taken and divided into two groups which were tested by the hematology analyzer. The control staining solution and staining solution B were mixed with the sample respectively, in a ratio of 4 μl of a sample to 1 ml of a staining solution. To samples, 0.01 g/L of surfactant N-alkyl betaine was added to spheroidize blood cells, and the temperature can be kept at about 42°C. The fluorescence intensity information (FL) of the cells of a treated blood sample was obtained by collecting side fluorescence at a detection angle of 90°, the side-scattered light intensity information (SS) of the cells of a treated blood sample was obtained by collecting side-scattered light at a detection angle of 90°, and the forward-scattered light intensity information (FS) of the cells in a treated blood sample was obtained by collecting forward-scattered light at a detection angle of 2°-5°, therefore a three-dimensional scatter diagram was obtained. Another 1 ml of the above-mentioned blood sample was taken, in which the platelet aggregation inducer ADP (at a final concentration of 0.01 mmol/L) was added, and the resulting solution was mixed evenly. Platelets were aggregated after 5 min. At this time, the sample was divided into two groups, which were tested by the hematology analyzer after being respectively treated with the control staining solution and staining solution B, and three-dimensional scatter diagrams were obtained. The scatter diagrams obtained by testing the fresh blood and the scatter diagrams obtained by testing the blood in which platelet aggregation was induced were as shown in FIG. 15.

As can be seen from FIG. 15, after the blood sample in which platelet aggregation was induced was treated with staining solution B for eliminating platelet aggregation interference, the aggregated platelets were disaggregated, and the detections of mature red blood cells, reticulocytes and platelets could be completed.

As shown in this figure, when the sample in which platelet aggregation was induced was treated with staining solution B, the fluorescence intensity (FL) of platelets in the three-dimensional scatter diagram was decreased, the forward-scattered intensity information (FS) was decreased, and the three-dimensional scatter diagram information obtained after disaggregation was close to the three-dimensional scatter diagram information of the blood sample with unaggregated platelets. In the scatter diagram using the control staining solution, the volume of a particle of aggregated platelets was larger than that of a single platelet, and the fluorescence intensity of the particle of aggregated platelets was also enhanced. In the three-dimensional scatter diagram, the proportion of the reticulocytes was calculated by calculating the percentage of the number of particles in the reticulocyte distribution region to the number of particles in the mature red blood cell and reticulocyte regions. The reticulocytes had a stronger fluorescent signal than normal red blood cells. The total red blood cell count in the unaggregated sample was determined as $3.94 \times 10^{12}$, in which the reticulocytes accounted for 1.96% in the total red blood cells. The total red blood cell count after disaggregation in the sample treated with staining agent B was determined as $3.97 \times 10^{12}$, indicating that the total red blood cell count was

not interfered by aggregated platelets. The platelet count in the unaggregated sample was determined as 242 × $10^9$; the platelet count in the aggregated sample was 23 × $10^9$; and the platelet count in the disaggregated sample was 207 × $10^9$.

Moreover, by counting the platelets, it was determined that the use of staining solution B increased the platelet count in the sample in which aggregation was induced, and the disaggregation rate reached 85.35%, whereas the disaggregation rate was 9.44% for the sample treated with the control straining solution.

**Test Example 2. Amino group-containing compound in diluent for detection of platelets and red blood cells**

This experimental example provided a diluent for the hematology analyzer, and the diluent can be used to eliminate platelet aggregation interference and used for detection of red blood cells and platelets. Such diluent had the formulations below.

Control diluent:

| | |
|---|---|
| citric acid | 0.5 g/L, |
| surfactant phenoxyethanol | 0.1 g/L, |
| bacteriostatic agent 1,3-dimethylurea | 6.0 g/L, |
| sodium chloride | 3.0 g/L. |

Diluent B:

1-acetylguanidine was added in the control diluent with the final concentration of 0.01 mol/L.

The above-mentioned diluents were prepared with distilled water at 25° C, with pH adjusted to 9.5 and the osmotic pressure of 200 mOsm/L.

The tests were performed according to a method similar to that in Test Example 1. The above-mentioned control diluent or diluent B was used, 50 mg/L of staining solution SYBR Green and 0.01 g/L of surfactant N-alkyl betaine were further added. The tests were performed to obtain three-dimensional scatter diagrams as shown in FIG. 16.

The total red blood cell count in the unaggregated sample was determined as 4.37 × $10^{12}$, where reticulocytes accounted for 1.44% in the total red blood cells, and the total red blood cell count after disaggregation was 4.30 × $10^{12}$, indicating that the total red blood cell count was not interfered by aggregated platelets. The platelet count in the unaggregated sample was determined as 186 × $10^9$; the platelet count in the aggregated sample was: 29 × $10^9$; and the platelet count in the disaggregated sample was 180 × $10^9$.

Moreover, by the platelet counts, it was determined that the use of diluent B increased the platelet count in the sample in which aggregation was induced and the disaggregation rate reached 96.32%, whereas the disaggregation rate was 15.67% for the control diluent.

**Test Example 3. Amino group-containing compound in combination with ammonium ion-containing compound in staining solution for detection of platelets and red blood cells**

This test example was implemented generally the same as in Test Example 1 except that staining solution C was used in place of staining solution B and the control staining solution was prepared according to the following composition.

Control staining solution:

| | |
|---|---|
| SYBR Green | 50 mg/L, |
| citric acid | 0.5 g/L, |
| surfactant phenoxyethanol | 0.1 g/L, |
| bacteriostatic agent 1,3-dimethylurea | 6.0 g/L, |
| sodium chloride | 1.0 g/L. |

Staining solution C:
to the control staining solution, 1-acetylguanidine was added with the final concentration of 0.01 mol/L and ammonium chloride was added with the final concentration be 0.01 mol/L.

Similarly, the staining solutions were prepared with distilled water at 25°C with pH adjusted to 9 and the osmotic pressure of 200 mOsm/L.

The tests were performed according to a method similar to Test Example 1 except that the final concentration of ADP was1 mol/L (100 times that in Test Example 1) to induce platelet aggregation in the sample. Three-dimensional scatter diagrams were obtained, as shown in FIG. 17.

The total red blood cell count in the unaggregated sample was determined as $6.14 \times 10^{12}$, where reticulocytes accounted for 1.34% in the total red blood cells, and the total red blood cell count in the sample in which platelet aggregation was induced and disaggregated was $6.11 \times 10^{12}$, indicating that the total red blood cell count was not interfered by aggregated platelets. The platelet count in the unaggregated sample was determined as $219 \times 10^9$; the platelet count in the aggregated sample was: $17 \times 10^9$; and the platelet count in the disaggregated sample was $182 \times 10^9$.

Moreover, by the platelet counts, it was determined that the use of diluent B increased the platelet count in the sample in which aggregation was induced and the disaggregation rate reached 82.73%, whereas the disaggregation rate was determined as only 7.61% for the control staining solution.

### Test Example 4. Amino group-containing compound in combination with ammonium ion-containing compound in diluent for detection of platelets and red blood cells

This test example was implemented generally the same as in Test Example 2 except that diluent B was replaced by diluent C. Diluent C was prepared by adding to the control diluent of 1-acetylguanidine with the final concentration of 0.01 mol/L and ammonium chloride with the final concentration remain 0.01 mol/L . The pH of each diluent was adjusted to 9.5, and the osmotic pressure was adjusted to 200 mOsm/L.

The tests were performed according to a method similar to Test Example 1 except that the final concentration of ADP was 1 mol/L (100 times that in Test Example 1) to induce platelet aggregation in the sample. Three-dimensional scatter diagrams were obtained, as shown in FIG. 18.

The total red blood cell count in the unaggregated sample was determined as $5.12 \times 10^{12}$, where reticulocytes accounted for 1.21% in the total red blood cells, and the total red blood cell count in the sample in which platelet aggregation was induced and disaggregated was $5.08 \times 10^{12}$, indicating that the total red blood cell count was not interfered by aggregated platelets. The platelet count in the unaggregated sample was determined as $278 \times 10^9$; the platelet count in the aggregated sample was: $18 \times 10^9$; and the platelet count in the disaggregated sample was $248 \times 10^9$.

Moreover, by the platelet counts, it was determined that the use of diluent B increased the platelet count in the sample in which aggregation was induced and the disaggregation rate reached 89.20%, whereas the disaggregation rate was 6.61% for the control diluent.

### Test Example 5. Amino group-containing compound in lytic buffer for detection of white blood cells

This test example provided a lytic buffer for the hematology analyzer, and the lytic buffer could be used for platelet disaggregation for detection of white blood cells. This test example and the following test examples all used the hematology analyzer (BC-6000Plus) from Shenzhen Mindray Bio-medical Electronics Co., Ltd. The lytic buffers were prepared according to the following formulations.

Control lytic buffer:

| | |
|---|---|
| hemolytic agent dimethylbenzylammonium chloride | 1.0 g/L |
| citric acid | 0.5 g/L, |
| surfactant phenoxyethanol | 0.1 g/L, |
| bacteriostatic agent 1,3-dimethylurea | 6.0 g/L, |
| sodium chloride | 3.0 g/L. |

Lytic buffer B:

1-acetylguanidine was added to make the final concentration be 0.01 mol/L on the basis of the control lytic buffer.

The control lytic buffer and lytic buffer B were prepared with distilled water at 25°C with pH adjusted to 9 and

the osmotic pressure of 90 mOsm/L.

According to the steps similar to those in Test Example 1, 1 ml of human venous blood sample collected into an anticoagulant tube was taken and divided into two groups, and the two groups were tested by using the hematology analyzer, where the control lytic buffer and lytic buffer B were mixed with the sample respectively, in a ratio of 20 μl of a sample to 1 ml of a lytic buffer. Then, 20 μl of a DNA staining agent (Hoechst33342) was added, and the temperature was kept at about 42°C. The fluorescence intensity information (FL) of the treated blood sample cells was determined by using side fluorescence at a detection angle of 90°, the side-scattered light intensity information (SS) of the treated blood sample cells was determined by using side-scattered light at a detection angle of 90°, and the forward-scattered light intensity information (FS) of the treated blood sample cells was determined by using forward-scattered light at a detection angle of 2°-5° therefor a three-dimensional scatter diagram was obtained. Another 1 ml of above-mentioned blood sample was taken, in which platelet aggregation inducer ADP (at a final concentration of 0.01 mmol/L) was added, and the resulting solution was mixed evenly. Platelets were aggregated after 5 min. At this time, the sample was divided into two groups, which were tested by the hematology analyzer after being treated with the control lytic buffer and lytic buffer B respectively, adding a DNA dye therein, and three-dimensional scatter diagrams were obtained. As above, 4 groups of scatter diagrams obtained from the sample test were as shown in FIG. 19.

In the optical channel, a plurality of aggregated platelets were recognized as one particle, and when the volume of the particle of the aggregated platelets is equal to the volume of white blood cells, the determination of the white blood cell count is influenced and the white blood cell count is increased. In this test example, the white blood cell count of the unaggregated sample was determined as $7.39 \times 10^9$, and the categories counts were $4.09 \times 10^9$ for neutrophils, $2.35 \times 10^9$ for lymphocytes, $0.32 \times 10^9$ for monocytes, and $0.52 \times 10^9$ for eosinophils. The white blood cell count of the sample after disaggregation by being treated with lytic buffer B was determined as $7.42 \times 10^9$, and the categories counts were $4.13 \times 10^9$ for neutrophils, $2.31 \times 10^9$ for lymphocytes, $0.34 \times 10^9$ for monocytes and $0.58 \times 10^9$ for eosinophils. The white blood cell count of the sample treated with the control lytic buffer after aggregation was determined as $8.02 \times 10^9$, and the categories counts were $4.37 \times 10^9$ for neutrophils, $2.56 \times 10^9$ for lymphocytes, $0.41 \times 10^9$ for monocytes and $0.63 \times 10^9$ for eosinophils. As can be seen, the count and classification of white blood cells were not influenced by platelet aggregation after lytic buffer B was used, and the count was more accurate.

### Test Example 6. Amino group-containing compound in combination with ammonium ion-containing compound in lytic buffer for detection of white blood cells

This test example performed the test according to the method in Test Example 5 except that lytic buffer C was prepared in place of lytic buffer B, that is, 1-acetylguanidine and $NH_4Cl$ at equal mole amounts were added to the control lytic buffer with the final concentration of 0.01 mol/L.

The control lytic buffer and lytic buffer C were prepared with distilled water at 25°C with pH adjusted to 9 and the osmotic pressure of 90 mOsm/L.

The test was performed according to the steps similar to those in Test Example 5, and three-dimensional scatter diagrams were obtained as shown in FIG. 20.

In this test example, the white blood cell count of the unaggregated sample was determined as $6.19 \times 10^9$, and the categories counts were $3.87 \times 10^9$ for neutrophils, $1.91 \times 10^9$ for lymphocytes, $0.22 \times 10^9$ for monocytes, and $0.08 \times 10^9$ for eosinophils. The white blood cell count of the sample after disaggregation by being treated with lytic buffer C was determined as $6.13 \times 10^9$, and the categories counts were $3.91 \times 10^9$ for neutrophils, $1.88 \times 10^9$ for lymphocytes, $0.21 \times 10^9$ for monocytes and $0.07 \times 10^9$ for eosinophils. The white blood cell count of the sample treated with the control lytic buffer after aggregation was determined as $6.97 \times 10^9$, and the categories counts were $4.21 \times 10^9$ for neutrophils, $2.21 \times 10^9$ for lymphocytes, $0.34 \times 10^9$ for monocytes and $0.17 \times 10^9$ for eosinophils. As can be seen, the count and classification of white blood cells were not influenced by platelet aggregation after lytic buffer C was used, and the count was more accurate.

### Test Example 7. Disaggregation effect of amino group-containing compound on platelet pseudo-aggregation in clinical practice

This test example related to the disaggregation of pseudo-aggregated platelets in clinical practice. The diluent same as diluent B in Test Example 1 was used for the test. The formulation of this diluent includes citric acid (0.5 g/L), surfactant phenoxyethanol (0.1 g/L), a bacteriostatic agent (6 g/L), sodium chloride (3 g/L) and 1-acetylguanidine (10 mmol/L). The pH of the diluent was adjusted to 9.5, and the osmotic pressure was adjusted to 200 mOsm/L.

The specific operations includes: collecting blood samples from outpatients and inpatients, and anti-coagulating with EDTA K2. Twenty samples meeting the EDTA-PTCP diagnostic standard were used as the experimental group, including 11 males and 9 females, with an average age of 56 years old, in which the PLT-I platelet counts were significantly reduced, and platelet aggregations were found by microscopic observation of blood smears, respectively. Blood samples

were drawn from patients whose blood showed platelet pseudo-aggregation without anticoagulant treatment; and then the blood was tested immediately on the hematology analyzer within 1 min (at this time, platelets were not aggregated), where the platelet count value determined by this method was the true value PLT_O (true value) of platelets. Blood samples drawn from the patients were collected into EDTA anticoagulant tubes. After 2 h (the platelets in samples from patients whose blood showed pseudo-aggregation were aggregated), the blood samples were tested by using the diluent containing 1-acetylguanidine as the disaggregation substance on the same hematology analyzer. The platelet count determined by this method was the disaggregation value PLT_O (disaggregated) of platelets. At the same time, blood samples were drawn from patients collected into EDTA anticoagulant tubes. After 2 h (the platelets of the samples from patients whose blood showed pseudo-aggregation were aggregated), the blood samples were tested by using the diluent free of the disaggregation substance 1-acetylguanidine on the same hematology analyzer. The platelet count determined by this method was the undisaggregation value PLT_O (undisaggregated) of platelets. The disaggregation effects were calculated by the equations:

$$\text{disaggregation rate (disaggregated)} = \text{PLT_O (disaggregated)/PLT_O (true value)};$$

or

$$\text{disaggregation rate (control)} = \text{PLT_O (undisaggregated)/PLT_O (true value)}.$$

The test result of each sample was shown in FIG. 21. As shown in FIG. 21, pseudo-aggregated platelets can be disaggregated to different degrees for all samples. Among 20 blood samples from the patients, 14 blood samples still had 70% disaggregation effect after left to stand for 2 h. In the control group, all of the disaggregation effects were below20%. The disaggregation diluent containing 10 mM 1-acetylguanidine had an obvious disaggregation effect.

For samples which are difficult to disaggregate, such as those having disaggregation rates of 70% or below, a desired disaggregation effect can be obtained by using a higher concentration, or by using a compound having a better disaggregation ability, or by using the combination of an amino group-containing compound and an ammonium ion-containing compound.

## Claims

**1.** Use of a composition for preventing and/or eliminating platelet aggregation in a blood sample in an *in vitro* blood test, wherein the composition comprises at least one compound selected from the group consisting of a compound of formula (I) and a salt thereof:

R1-NH-R2 (I)

wherein R1 and R2 are same or different and are each independently selected from the group consisting of H, $-SO_3H$, $-NH_2$, $-C(NH)-NH_2$, substituted or unsubstituted C1-16 alkyl, substituted or unsubstituted C6-10 aryl, substituted or unsubstituted C7-14 alkaryl, substituted or unsubstituted C7-14 aralkyl, -C(O)-Q1 and -C(O)-O-Q2, provided that R1 and R2 are not H simultaneously,

wherein Q1 is H, $-NH_2$, substituted or unsubstituted C1-16 alkyl, substituted or unsubstituted C6-10 aryl, substituted or unsubstituted C7-14 alkaryl or substituted or unsubstituted C7-14 aralkyl, and

Q2 is H, substituted or unsubstituted C1-16 alkyl, substituted or unsubstituted C6-10 aryl, substituted or unsubstituted C7-14 alkaryl or substituted or unsubstituted C7-14 aralkyl,

wherein "substituted" means being substituted with at least one selected from the group consisting of -NP1P2, $-SO_3H$, -OH, halogen, -CN, -C(O)-O-P3, -O-C1-16 alkyl, -O-C6-10 aryl, -O-C7-14 alkaryl, -O-C7-14 aralkyl, -C(O)-C1-16 alkyl, -C(O)-C6-10 aryl, -C(O)-C7-14 alkaryl, -C(O)-C7-14 aralkyl and -C(O)-NP1P2, wherein -O-C1-16 alkyl, -O-C6-10 aryl, -O-C7-14 alkaryl, -O-C7-14 aralkyl, -C(O)-C1-16 alkyl, -C(O)-C6-10 aryl, -C(O)-C7-14 alkaryl and -C(O)-C7-14 aralkyl are unsubstituted or further substituted with at least one selected from the group consisting of $-NH_2$, -OH, $-SO_3H$, halogen, -CN, -COOH and $-C(O)NH_2$, respectively, and

P1, P2 and P3 are each independently selected from the group consisting of H, C1-16 alkyl, C6-10 aryl, C7-14 alkaryl and C7-14 aralkyl, wherein C1-16 alkyl, C6-10 aryl, C7-14 alkaryl and C7-14 aralkyl are unsubstituted or further substituted with at least one selected from the group consisting of $-NH_2$, -OH, $-SO_3H$, halogen, -CN, -COOH and $-C(O)NH_2$, respectively.

2. The use of claim 1, wherein in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-Q2, provided that R1 and R2 are not H simultaneously,

   wherein Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl, and
   Q2 is H, substituted or unsubstituted C1-10 alkyl, substituted or unsubstituted C6-10 aryl, substituted or unsubstituted C7-10 alkaryl or substituted or unsubstituted C7-10 aralkyl,
   wherein "substituted" means being substituted with at least one selected from the group consisting of -NP1P2, $-SO_3H$, -OH, -CN, -C(O)-O-P3, -O-C1-10 alkyl, -O-C6-10 aryl, -O-C7-10 alkaryl, -O-C7-10 aralkyl and -C(O)-NP1P2, wherein -O-C1-10 alkyl, -O-C6-10 aryl, -O-C7-10 alkaryl and -O-C7-10 aralkyl are unsubstituted or further substituted with at least one selected from the group consisting of $-NH_2$, -OH, - $SO_3H$, -CN, -COOH and $-C(O)NH_2$, respectively, and
   P1, P2 and P3 are each independently selected from: H, C1-10 alkyl, C6-10 aryl, C7-10 alkaryl and C7-10 aralkyl, wherein C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and $-C(O)NH_2$, respectively;
   preferably, in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-H, provided that R1 and R2 are not H simultaneously,
   wherein Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl, and
   wherein "substituted" means being substituted with at least one selected from the group consisting of -NP1P2, $-SO_3H$, -OH, -CN, -C(O)-O-H and -C(O)-NP1P2, and
   P1 and P2 are each independently selected from the group consisting of H, C1-10 alkyl, C6-10 aryl, C7-10 alkaryl and C7-10 aralkyl, wherein C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and - $C(O)NH_2$, respectively.

3. The use of claim 1, wherein the compound of formula (I) has a total of 1-20 primary amino groups, secondary amino groups and/or imino groups.

4. The use of claim 1, wherein preventing and/or eliminating the platelet aggregation in the blood sample is performed in a solution having a pH value of at least 3.0, preferably having a pH value of 7.5-11.

5. The use of claim 4, wherein an amino group of the compound of formula (I) has a pKa value of 1-16, preferably of 4-14, and more preferably the pKa value is less than the pH value.

6. The use of claim 4, wherein the compound of formula (I) has a concentration of 1-50 mmol/L, preferably 2-20 mmol/L, in the solution.

7. The use of claim 1, wherein the composition further comprises at least one ammonium ion-containing compound; preferably, the ammonium ion-containing compound contains an anion selected from the group consisting of a chloride ion, a bromide ion, an iodide ion, hydroxide, phosphate, hydrogen phosphate, dihydrogen phosphate, nitrate, hydrosulfide, thiocyanate, sulfate, bisulfate, sulfite, bisulfite, carbonate, bicarbonate, formate, acetate, oxalate, propionate, malonate, citrate and a combination thereof; and more preferably, the ammonium salt is at least one selected from ammonium chloride, ammonium bromide, ammonium iodide, ammonium phosphate, ammonium hydrogen phosphate, ammonium dihydrogen phosphate, ammonium nitrate, ammonium thiocyanate, ammonium bisulfite, ammonium oxalate, ammonium hydroxide, ammonium bisulfate and ammonium bicarbonate.

8. The use of claim 7, wherein the ammonium ion-containing compound has a concentration of 1-50 mmol/L, preferably 2-20 mmol/L, in preventing and/or eliminating the platelet aggregation in the blood sample.

9. The use of any one of claims 1-8, wherein the *in vitro* blood test comprises at least one of platelet detection, red blood cell detection and white blood cell detection.

**10.** The use of any one of claims 1-8, wherein the blood sample is taken from peripheral blood or venous blood in a mammal.

**11.** Use of a composition for preventing and/or eliminating platelet aggregation in a blood sample in an *in vitro* blood test, wherein the composition comprises at least one amino group-containing compound, and in a solution for preventing and/or eliminating the platelet aggregation in the blood sample, the amino group of the amino group-containing compound has a pKa value less than or equal to a pH value of the solution.

**12.** The use of claim 11, wherein the amino group of the amino group-containing compound has the pKa value of 1-14; preferably, the amino group-containing compound has at least one of primary amino group and secondary amino group; and more preferably, the amino group-containing compound has a total of 1-20 primary amino groups, secondary amino groups and/or imino groups.

**13.** The use of claim 11, wherein the pH value of the solution is at least 3.0, preferably 7.5-11.

**14.** The use of claim 11, wherein the amino group-containing compound is selected from the group consisting of a compound of formula (I) and a salt thereof:

R1-NH-R2 (I)

wherein the compound of formula (I) is as defined in claim 1 or 2.

**15.** The use of claim 11, wherein the amino group-containing compound has a concentration of 1-50 mmol/L, preferably 2-20 mmol/L in the solution.

**16.** The use of claim 11, wherein the composition further comprises at least one ammonium ion-containing compound; and preferably the ammonium ion-containing compound is as defined in claim 7.

**17.** The use of any one of claim 16, wherein the ammonium ion-containing compound has a concentration of 1-50 mmol/L, preferably 2-20 mmol/L, in preventing and/or eliminating the platelet aggregation in the blood sample.

**18.** An agent for reducing platelet aggregation interference in an *in vitro* blood test, wherein the agent comprises at least one of a compound of formula (I) and a salt thereof at a concentration of 1-50 mmol/L:

R1-NH-R2 (I)

wherein R1 and R2 are same or different and are each independently selected from the group consisting of H, $-SO_3H$, $-NH_2$, $-C(NH)-NH_2$, substituted or unsubstituted C1-16 alkyl, substituted or unsubstituted C6-10 aryl, substituted or unsubstituted C7-14 alkaryl, substituted or unsubstituted C7-14 aralkyl, -C(O)-Q1 and -C(O)-O-Q2, provided that R1 and R2 are not H simultaneously,
wherein Q1 is H, $-NH_2$, substituted or unsubstituted C1-16 alkyl, substituted or unsubstituted C6-10 aryl, substituted or unsubstituted C7-14 alkaryl or substituted or unsubstituted C7-14 aralkyl, and
Q2 is H, substituted or unsubstituted C1-16 alkyl, substituted or unsubstituted C6-10 aryl, substituted or unsubstituted C7-14 alkaryl or substituted or unsubstituted C7-14 aralkyl,
wherein "substituted" means being substituted with at least one selected from the group consisting of -NP1P2, $-SO_3H$, -OH, halogen, -CN, -C(O)-O-P3, -O-C1-16 alkyl, -O-C6-10 aryl, -O-C7-14 alkaryl, -O-C7-14 aralkyl, -C(O)-C1-16 alkyl, -C(O)-C6-10 aryl, -C(O)-C7-14 alkaryl, -C(O)-C7-14 aralkyl and -C(O)-NP1P2, wherein -O-C1-16 alkyl, -O-C6-10 aryl, -O-C7-14 alkaryl, -O-C7-14 aralkyl, -C(O)-C1-16 alkyl, -C(O)-C6-10 aryl, -C(O)-C7-14 alkaryl and -C(O)-C7-14 aralkyl are unsubstituted or further substituted with at least one selected from the group consisting of $-NH_2$, -OH, $-SO_3H$, halogen, -CN, -COOH and $-C(O)NH_2$, respectively, and
P1, P2 and P3 are each independently selected from: H, C1-16 alkyl, C6-10 aryl, C7-14 alkaryl and C7-14 aralkyl, wherein C1-16 alkyl, C6-10 aryl, C7-14 alkaryl and C7-14 aralkyl are unsubstituted or further substituted with at least one selected from the group consisting of $-NH_2$, -OH, $-SO_3H$, halogen, -CN, -COOH and $-C(O)NH_2$, respectively.

**19.** The agent for reducing the platelet aggregation interference of claim 18, wherein in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-Q2, provided that R1 and R2 are not H simultaneously,

wherein Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl, and
Q2 is H, substituted or unsubstituted C1-10 alkyl, substituted or unsubstituted C6-10 aryl, substituted or unsubstituted C7-10 alkaryl or substituted or unsubstituted C7-10 aralkyl,
wherein "substituted" means being substituted with at least one selected from the group consisting of -NP1P2, $-SO_3H$, -OH, -CN, -C(O)-O-P3, -O-C1-10 alkyl, -O-C6-10 aryl, -O-C7-10 alkaryl, -O-C7-10 aralkyl and -C(O)-NP1P2, wherein the -O-C1-10 alkyl, the -O-C6-10 aryl, the -O-C7-10 alkaryl and the -O-C7-10 aralkyl are unsubstituted or further substituted with at least one selected from the group consisting of $-NH_2$, -OH, $-SO_3H$, -CN, -COOH and $-C(O)NH_2$, respectively, and
P1, P2 and P3 are each independently selected from: H, C1-10 alkyl, C6-10 aryl, C7-10 alkaryl and C7-10 aralkyl, wherein C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and $-C(O)NH_2$, respectively;
preferably, in the compound of formula (I), R1 and R2 are same or different and are each independently selected from the group consisting of H, $-SO_3H$, $-NH_2$, $-C(NH)-NH_2$, substituted or unsubstituted C1-10 alkyl, substituted or unsubstituted C6-10 aryl, substituted or unsubstituted C7-10 alkaryl, substituted or unsubstituted C7-10 aralkyl, -C(O)-Q1 and -C(O)-O-H, provided that R1 and R2 are not H simultaneously,
wherein Q1 is H, $-NH_2$, substituted or unsubstituted C1-10 alkyl, substituted or unsubstituted C6-10 aryl, substituted or unsubstituted C7-10 alkaryl or substituted or unsubstituted C7-10 aralkyl, and
wherein "substituted" means being substituted with at least one selected from the group consisting of -NP1P2, $-SO_3H$, -OH, -CN, -C(O)-O-H and -C(O)-NP1P2, and
P1 and P2 are each independently selected fromthe group consisting of H, C1-10 alkyl, C6-10 aryl, C7-10 alkaryl and C7-10 aralkyl, wherein C1-10 alkyl, C6-10 aryl, C7-10 alkaryl and C7-10 aralkyl are unsubstituted or further substituted with at least one selected from the group consisting of $-NH_2$, -OH, $-SO_3H$, -CN, -COOH and - $C(O)NH_2$, respectively.

**20.** The agent for reducing the platelet aggregation interference of claim 18, wherein the agent has a pH value of at least 3.0, preferably 7.5-11.

**21.** The agent for reducing the platelet aggregation interference of claim 18, wherein an amino group of the compound of formula (I) has a pKa value of 1-16, preferably 4-14, more preferably the pKa value is less than or equal to the pH value of the solution.

**22.** The agent for reducing the platelet aggregation interference of claim 18, wherein the compound of formula (I) has a concentration of 2-20 mmol/L.

**23.** The agent for reducing the platelet aggregation interference of claim 18, wherein the agent further comprises at least one ammonium ion-containing compound having a concentration of 1-50 mmol/L, preferably 2-20 mmol/L; preferably, the ammonium ion-containing compound contains an anion selected from the group consisting of chloride ion, bromide ion, iodide ion, hydroxide, phosphate, hydrogen phosphate, dihydrogen phosphate, nitrate, hydrosulfide, thiocyanate, sulfate, bisulfate, sulfite, bisulfite, carbonate, bicarbonate, formate, acetate, oxalate, propionate, malonate and citrate and a combination thereof; and more preferably, the ammonium ion-containing compound is selected from at least one of ammonium chloride, ammonium bromide, ammonium iodide, ammonium phosphate, ammonium hydrogen phosphate, ammonium dihydrogen phosphate, ammonium nitrate, ammonium thiocyanate, ammonium bisulfite, ammonium oxalate, ammonium hydroxide, ammonium bisulfate and ammonium bicarbonate.

**24.** The agent for reducing the platelet aggregation interference of any one of claims 18-23, wherein the agent further comprises a buffer, an osmotic pressure regulator and optionally at least one selected from a surfactant, a fluorescent dye and a red blood cell lytic agent.

**25.** A method for preventing and/or eliminating platelet aggregation in a sample in an *in vitro* blood test, comprising treating a blood sample with the composition as defined in any one of claims 1-17 or with the agent of any one of claims 18-24.

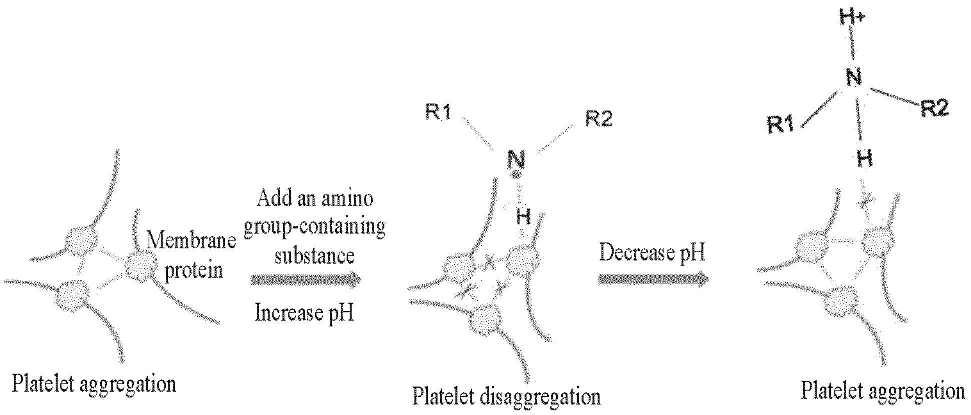


*FIG. 1*

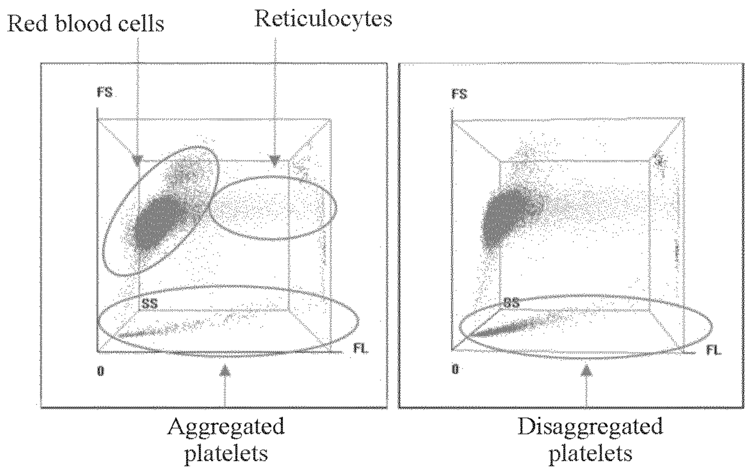


*FIG. 2*

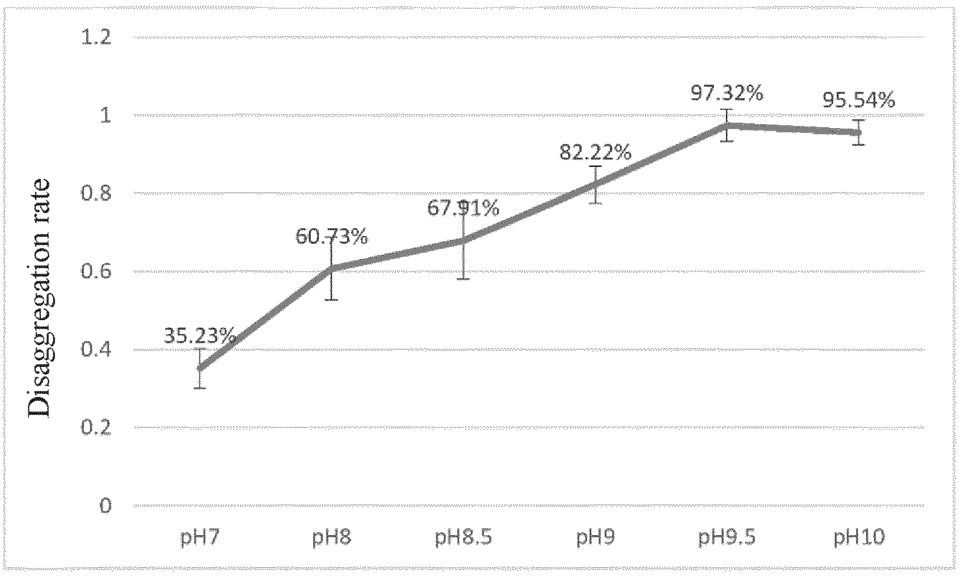


*FIG. 3*

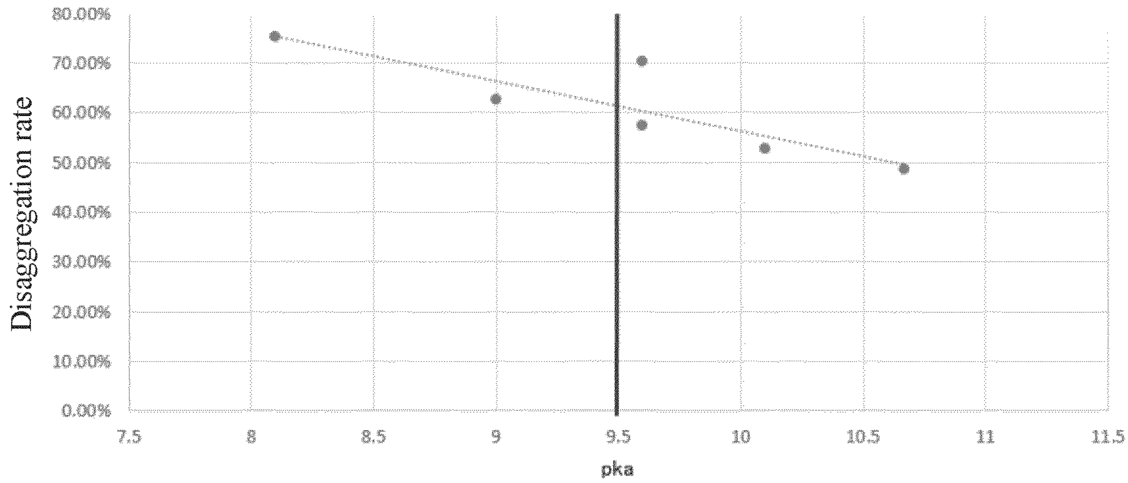
Disaggregation rate
80.00%
70.00%
60.00%
50.00%
40.00%
30.00%
20.00%
10.00%
0.00%
7.5
8
8.5
9
9.5
10
10.5
11
11.5
pka

*FIG. 4*

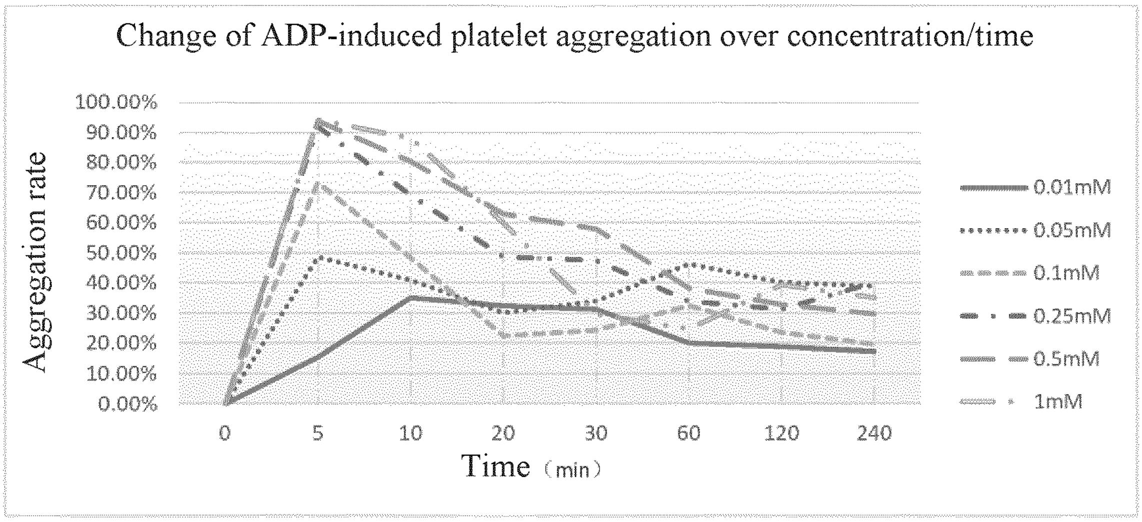
Change of ADP-induced platelet aggregation over concentration/time
Aggregation rate
100.00%
90.00%
80.00%
70.00%
60.00%
50.00%
40.00%
30.00%
20.00%
10.00%
0.00%
0
5
10
20
30
60
120
240
Time（min）
0.01mM
0.05mM
0.1mM
0.25mM
0.5mM
1mM

*FIG. 5*

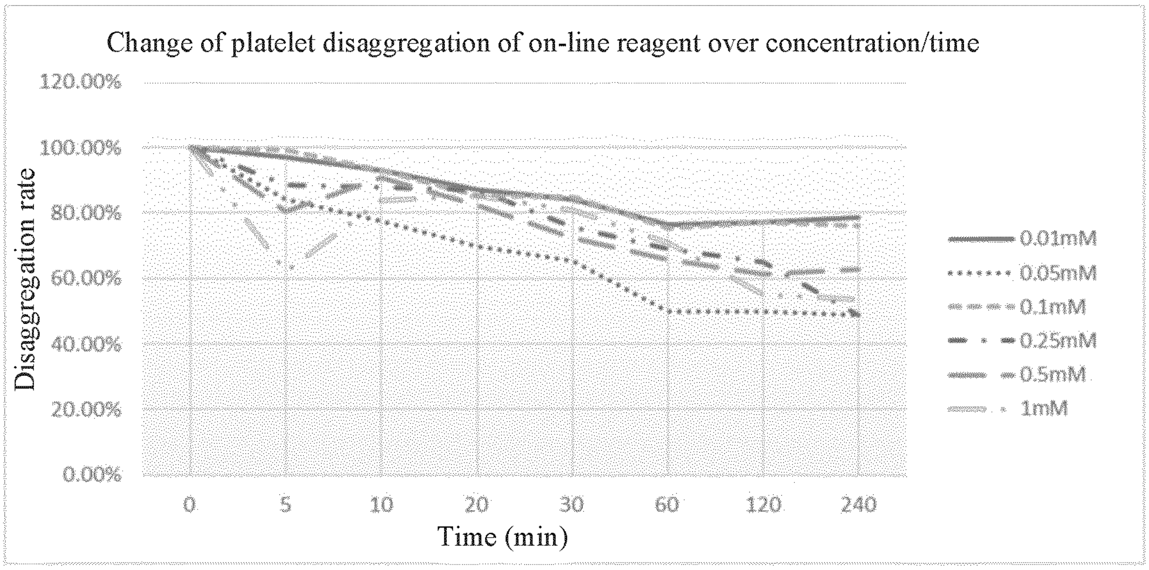
Change of platelet disaggregation of on-line reagent over concentration/time
Disaggregation rate
120.00%
100.00%
80.00%
60.00%
40.00%
20.00%
0.00%
0
5
10
20
30
60
120
240
Time (min)
0.01mM
0.05mM
0.1mM
0.25mM
0.5mM
1mM

*FIG. 6*

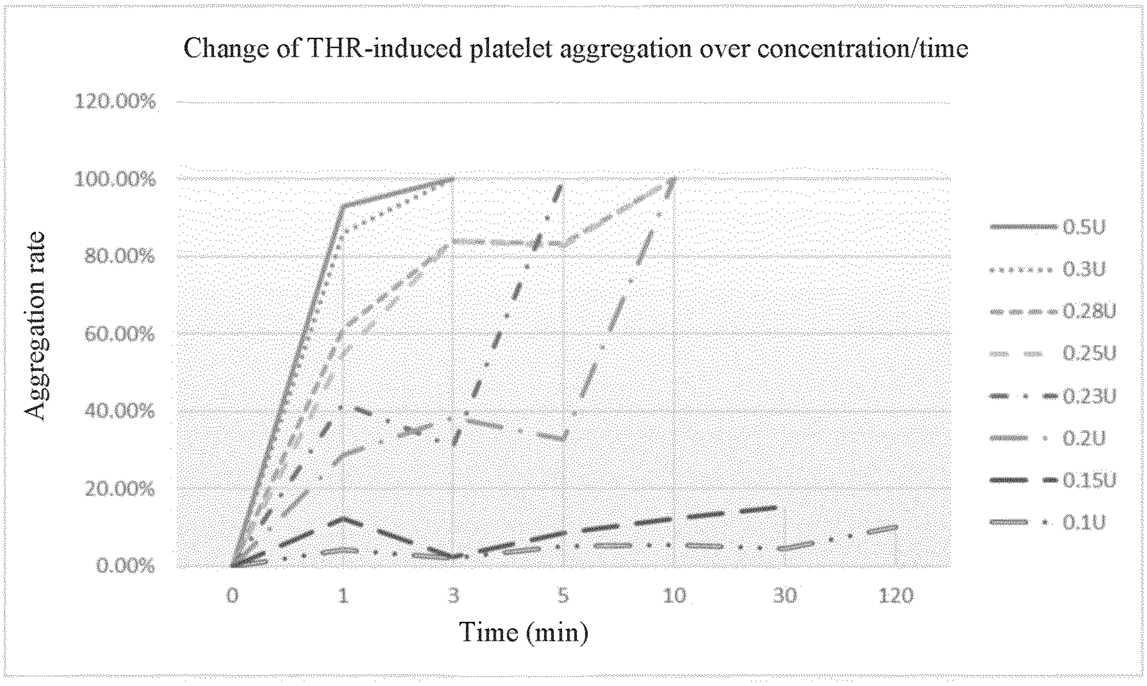
Change of THR-induced platelet aggregation over concentration/time
Aggregation rate
120.00%
100.00%
80.00%
60.00%
40.00%
20.00%
0.00%
0
1
3
5
10
30
120
Time (min)
0.5U
0.3U
0.28U
0.25U
0.23U
0.2U
0.15U
0.1U

*FIG. 7*

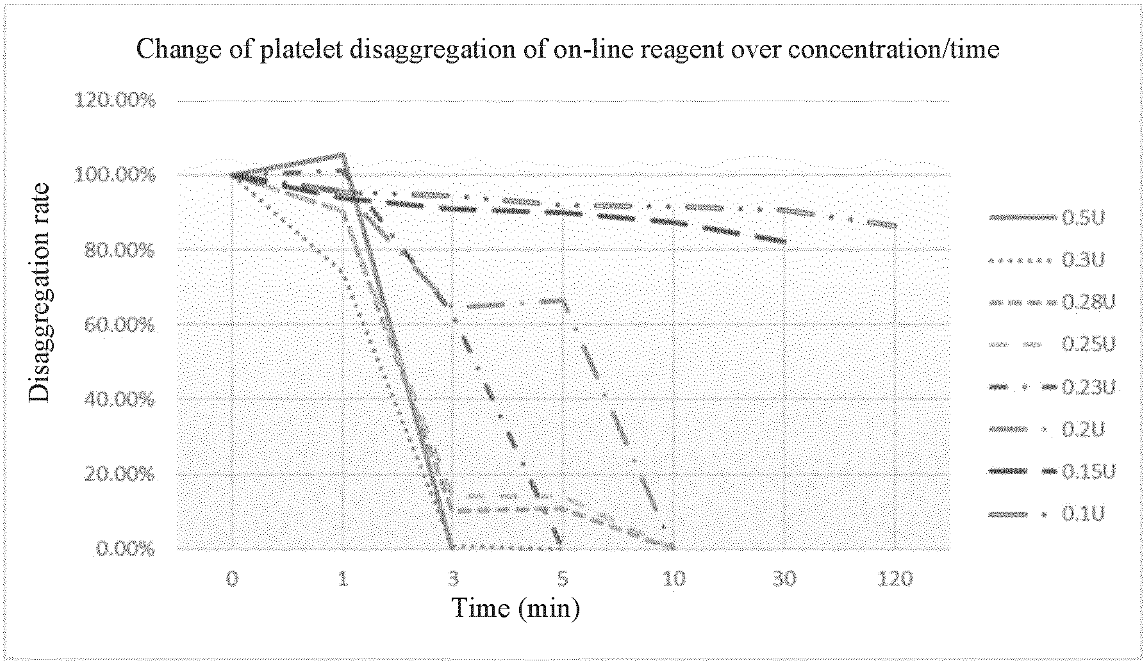
Change of platelet disaggregation of on-line reagent over concentration/time
Disaggregation rate
120.00%
100.00%
80.00%
60.00%
40.00%
20.00%
0.00%
0
1
3
5
10
30
120
Time (min)
0.5U
0.3U
0.28U
0.25U
0.23U
0.2U
0.15U
0.1U

*FIG. 8*

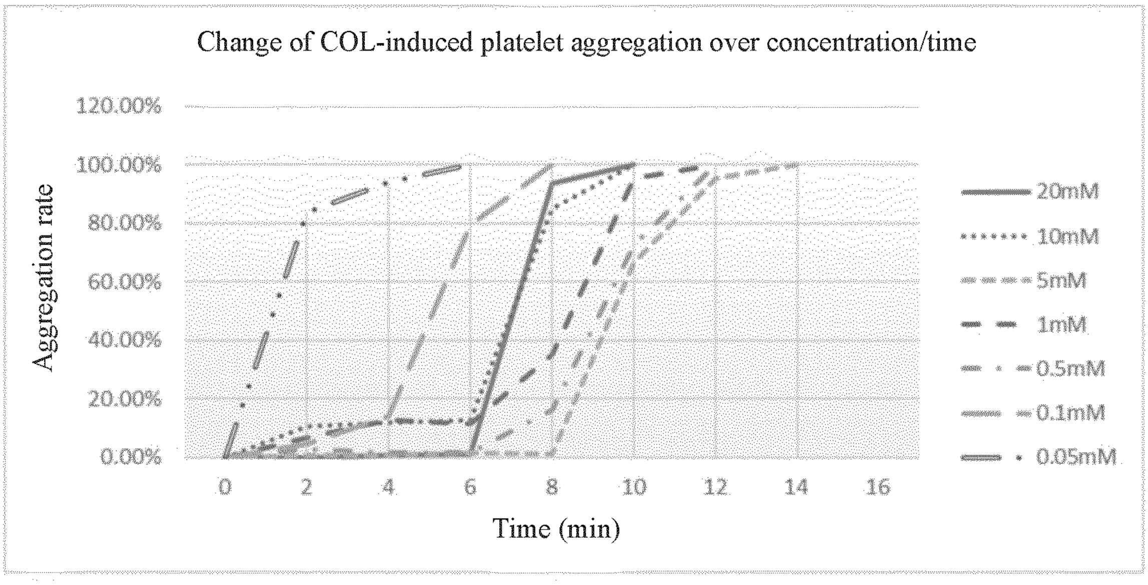
Change of COL-induced platelet aggregation over concentration/time
Aggregation rate
120.00%
100.00%
80.00%
60.00%
40.00%
20.00%
0.00%
0
2
4
6
8
10
12
14
16
Time (min)
20mM
10mM
5mM
1mM
0.5mM
0.1mM
0.05mM

*FIG. 9*

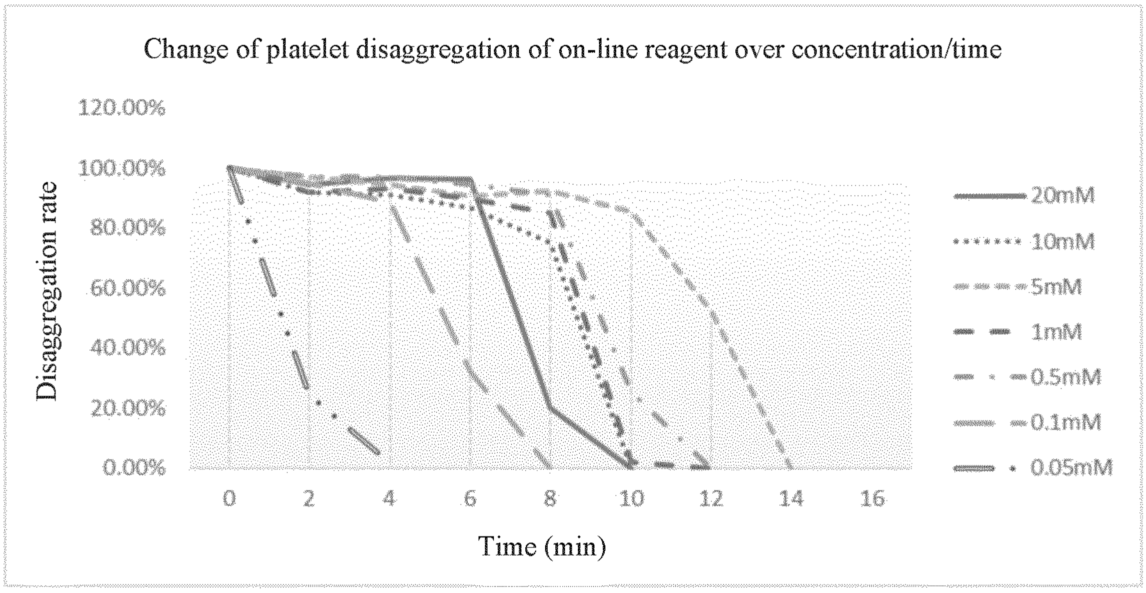
Change of platelet disaggregation of on-line reagent over concentration/time
Disaggregation rate
120.00%
100.00%
80.00%
60.00%
40.00%
20.00%
0.00%
0
2
4
6
8
10
12
14
16
Time (min)
20mM
10mM
5mM
1mM
0.5mM
0.1mM
0.05mM

*FIG. 10*

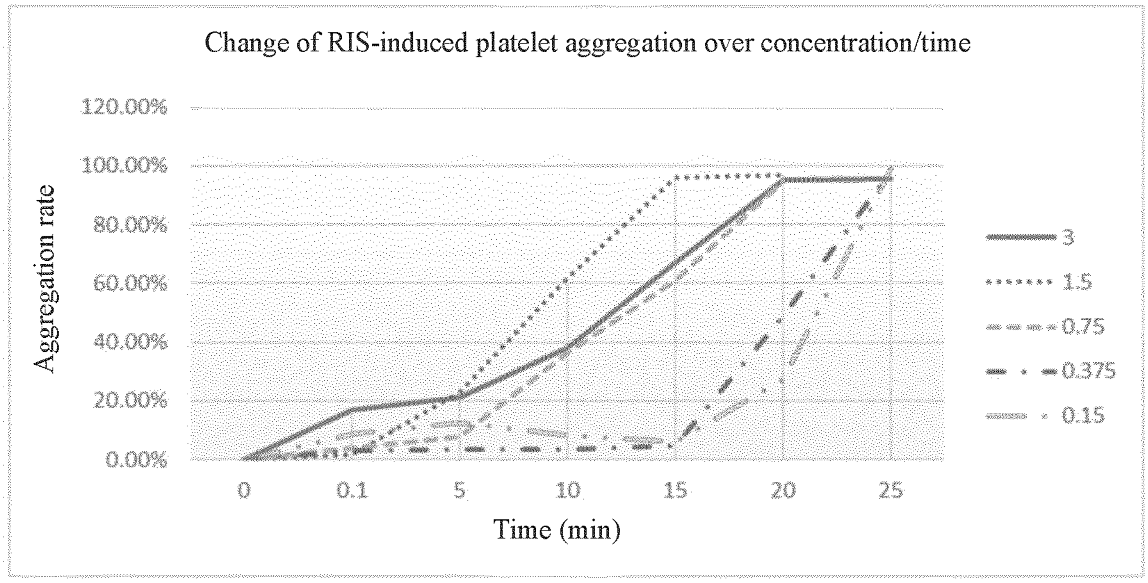
Change of RIS-induced platelet aggregation over concentration/time
Aggregation rate
120.00%
100.00%
80.00%
60.00%
40.00%
20.00%
0.00%
0
0.1
5
10
15
20
25
Time (min)
3
1.5
0.75
0.375
0.15

*FIG. 11*

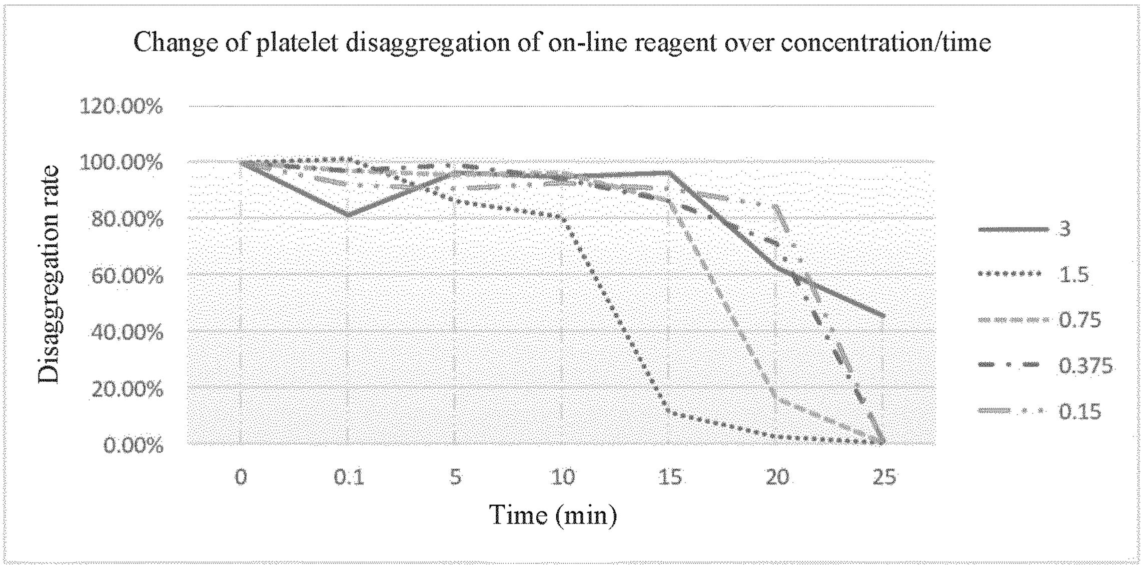

Change of platelet disaggregation of on-line reagent over concentration/time
Disaggregation rate
120.00%
100.00%
80.00%
60.00%
40.00%
20.00%
0.00%
0
0.1
5
10
15
20
25
Time (min)
3
1.5
0.75
0.375
0.15


*FIG. 12*

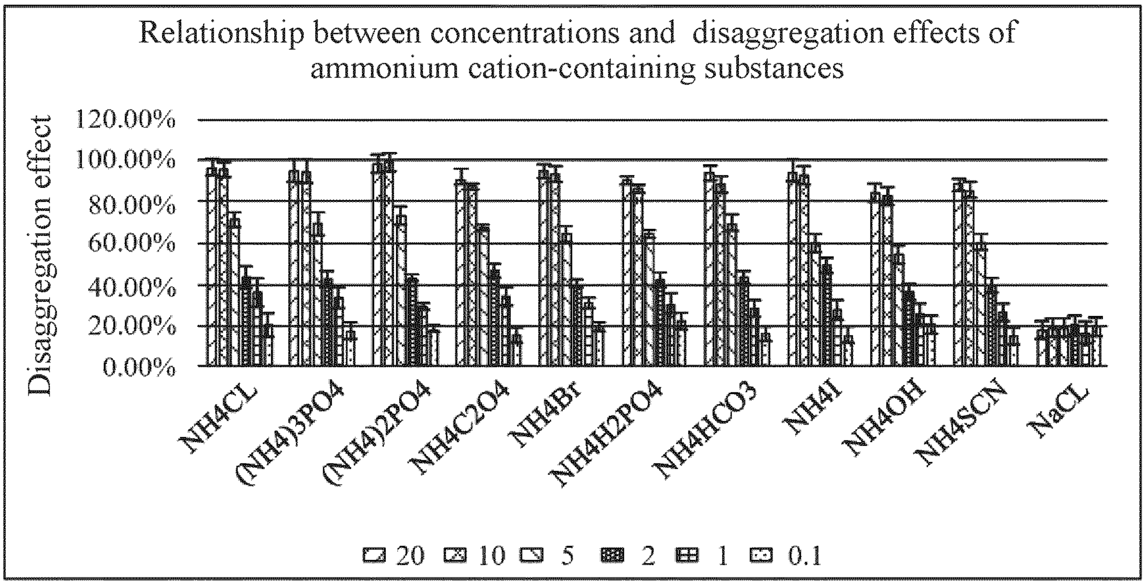

Relationship between concentrations and disaggregation effects of ammonium cation-containing substances
Disaggregation effect
120.00%
100.00%
80.00%
60.00%
40.00%
20.00%
0.00%
NH4CL
(NH4)3PO4
(NH4)2PO4
NH4C2O4
NH4Br
NH4H2PO4
NH4HCO3
NH4I
NH4OH
NH4SCN
NaCL
20
10
5
2
1
0.1


*FIG. 13*

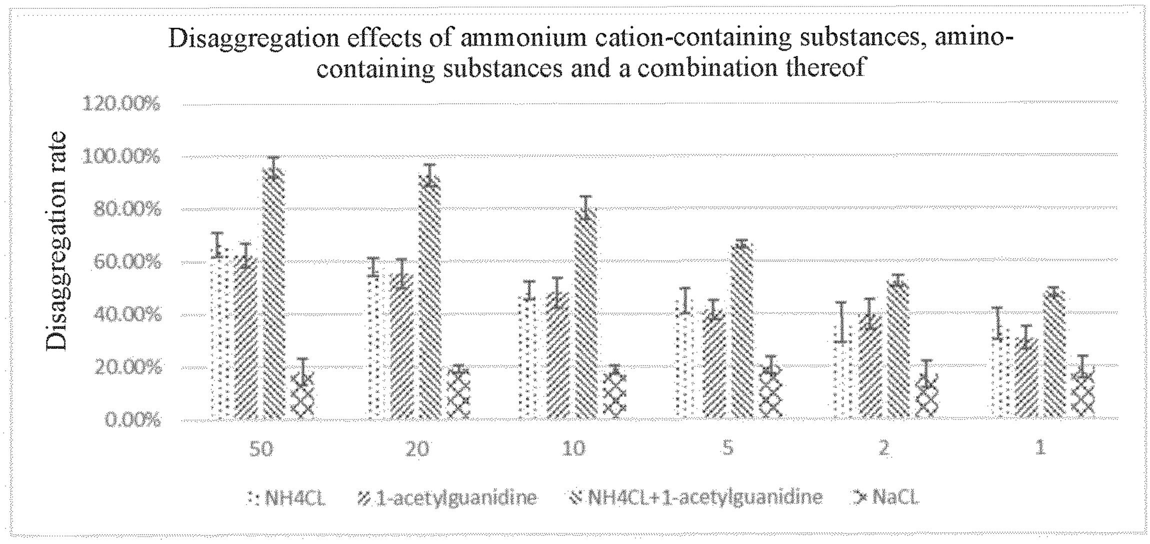


*FIG. 14*

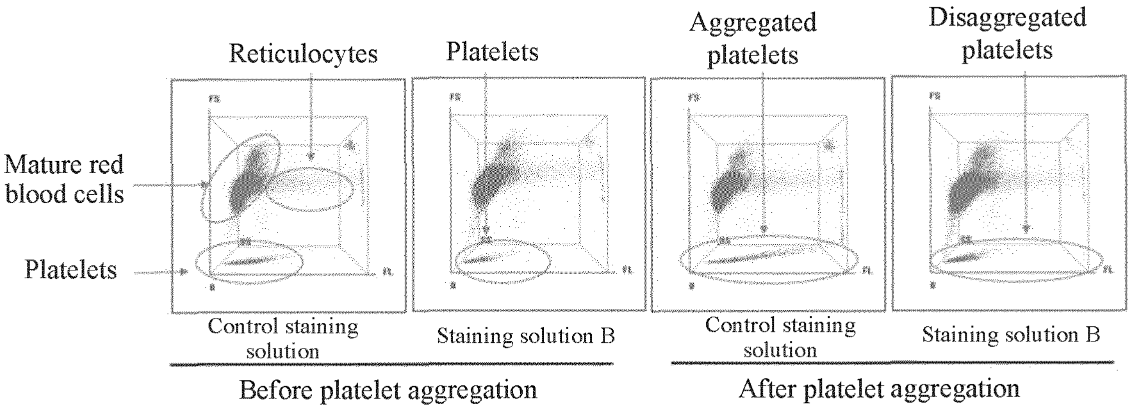


*FIG. 15*

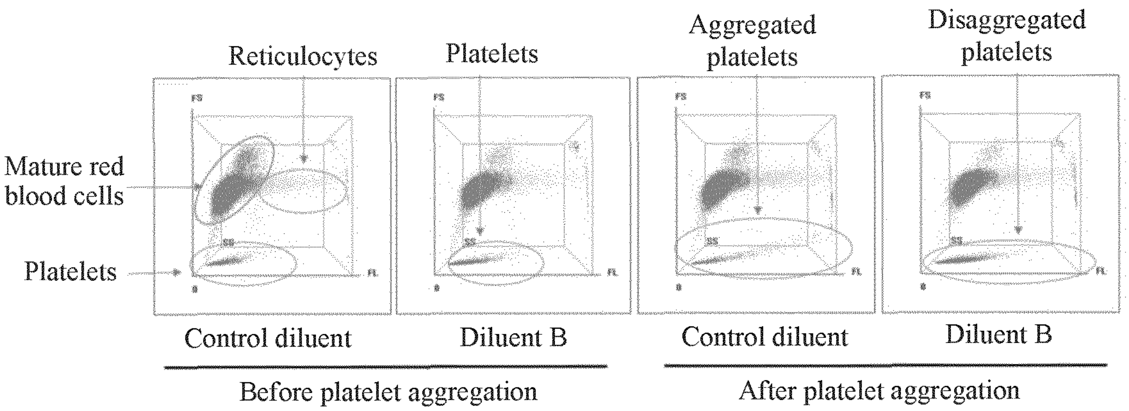


*FIG. 16*

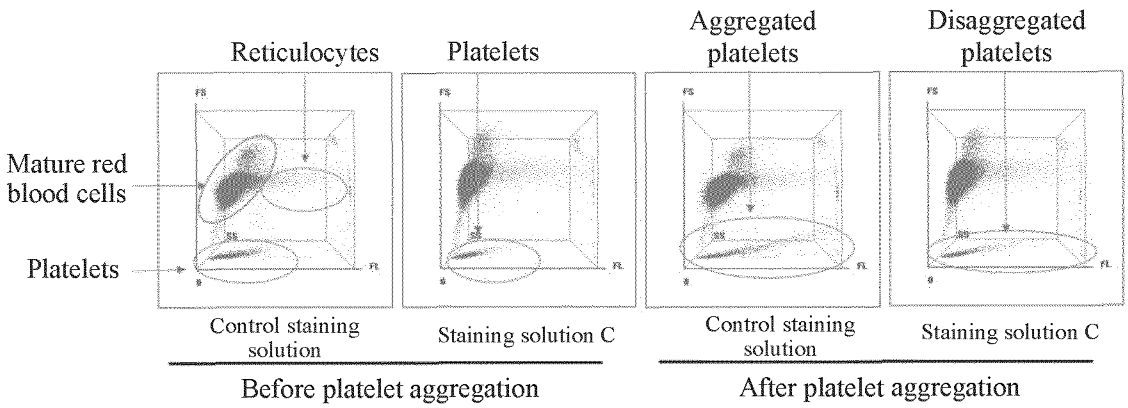


*FIG. 17*

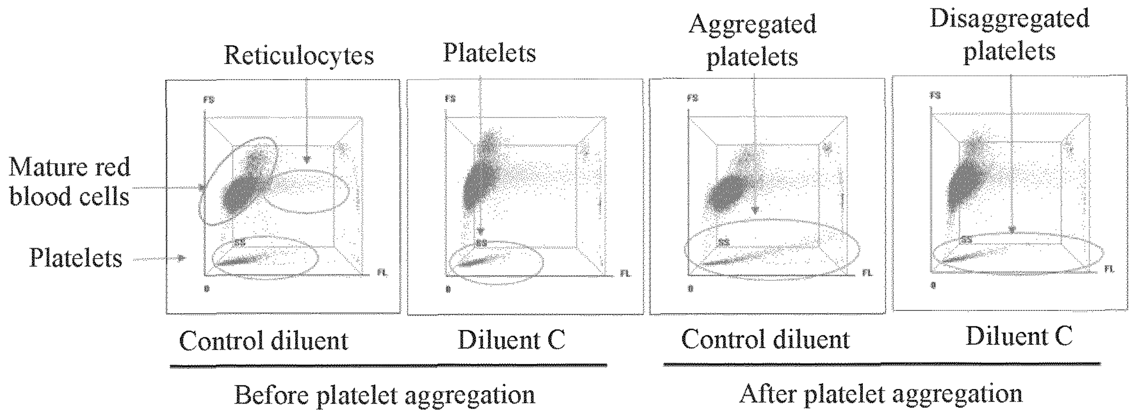


*FIG. 18*

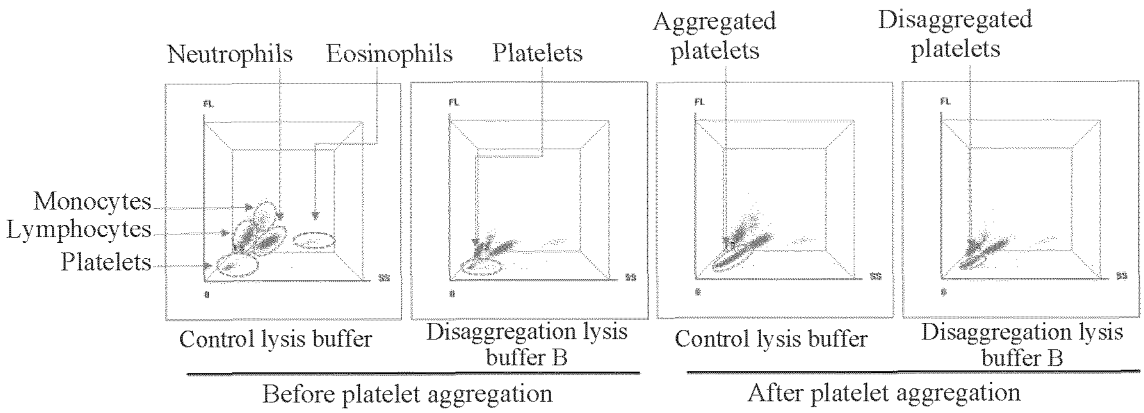


*FIG. 19*

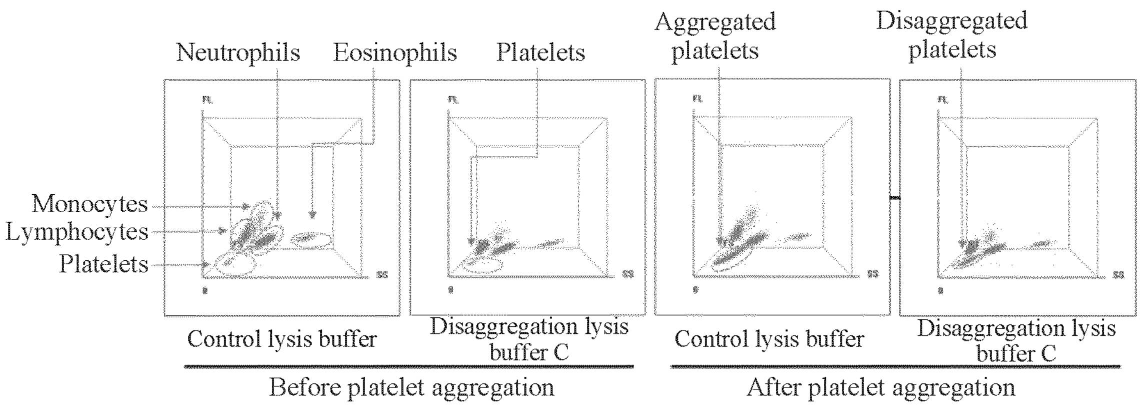
Neutrophils
Eosinophils
Platelets
Aggregated platelets
Disaggregated platelets
Monocytes
Lymphocytes
Platelets
Control lysis buffer
Disaggregation lysis buffer C
Control lysis buffer
Disaggregation lysis buffer C
Before platelet aggregation
After platelet aggregation

*FIG. 20*

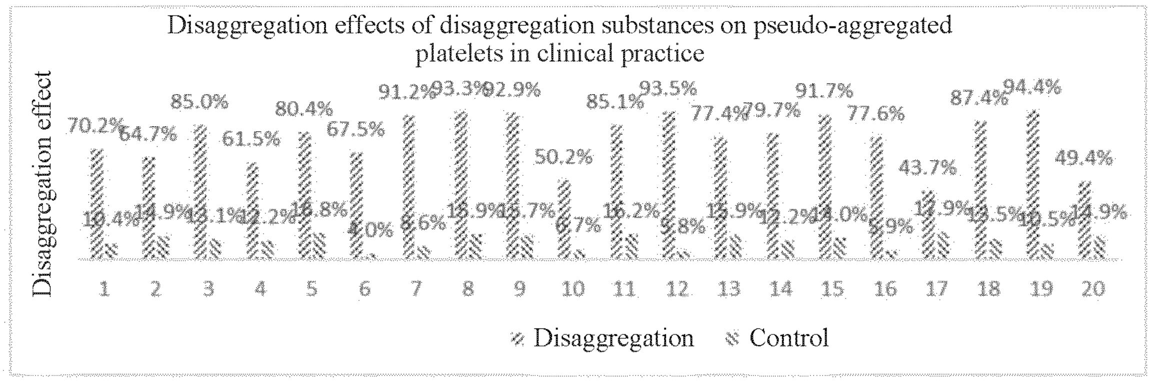
Disaggregation effects of disaggregation substances on pseudo-aggregated platelets in clinical practice
Disaggregation effect
70.2% 64.7% 85.0% 61.5% 80.4% 67.5% 91.2% 93.3% 92.9% 50.2% 85.1% 93.5% 77.4% 79.7% 91.7% 77.6% 43.7% 87.4% 94.4% 49.4%
10.4% 14.9% 13.1% 12.2% 18.8% 4.0% 6.6% 15.9% 15.7% 6.7% 16.2% 5.8% 15.9% 12.2% 14.0% 5.9% 17.9% 13.5% 10.5% 14.9%
1 2 3 4 5 6 7 8 9 10 11 12 13 14 15 16 17 18 19 20
Disaggregation
Control

*FIG. 21*

**INTERNATIONAL SEARCH REPORT**

International application No.
**PCT/CN2020/103136**

**A. CLASSIFICATION OF SUBJECT MATTER**

C07D 257/04(2006.01)i; C07D 317/46(2006.01)i; C07D 405/12(2006.01)i; C07D 237/04(2006.01)i; A61K 31/50(2006.01)i; A61K 31/41(2006.01)i; A61K 31/36(2006.01)i; A61P 7/02(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNABS, CNMED, CNTXT, TWTXT, DWPI, SIPOABS, USTXT, EPTXT, WOTXT, CNKI, 中国药物专利数据库 CTCMPTD, 中国药学文摘, 百度学术搜索, ISI-WEB OF SCIENCE, STN: 氨基, 血小板, 血栓, 体外, amino, blood platelet, thrombus, vitro

**C. DOCUMENTS CONSIDERED TO BE RELEVANT**

| Category* | Citation of document, with indication, where appropriate, of the relevant passages | Relevant to claim No. |
|---|---|---|
| X | 周远鹏 等 (ZHOU, Yuanpeng et al.). "益母草碱、丁香酰胍醇及丁香酸氨基醇酯类化合物抗血小板聚集活性及其与结构的关系 (Structure-Activity Relationship of Leonurine, Syringy 4-Hydroxybutunidine and Syringic Acid Aminoesters in Inhibiting Platelet Aggregation)" *中国药学杂志 (Chinese Pharmaceutical Journal),* Vol. 31, No. 5, 31 May 1996 (1996-05-31), abstract, page 273, Conclusions | 1, 3, 5, 9, 11-12, 14, 19, 25 |
| Y | 周远鹏 等 (ZHOU, Yuanpeng et al.). "益母草碱、丁香酰胍醇及丁香酸氨基醇酯类化合物抗血小板聚集活性及其与结构的关系 (Structure-Activity Relationship of Leonurine, Syringy 4-Hydroxybutunidine and Syringic Acid Aminoesters in Inhibiting Platelet Aggregation)" *中国药学杂志 (Chinese Pharmaceutical Journal),* Vol. 31, No. 5, 31 May 1996 (1996-05-31), abstract, page 273, Conclusions | 7-8, 16-17, 23 |
| X | 许勤龙 等 (XU, Qinlong et al.). "N-(4-胍基丁基)丁香酰胺对家兔血小板聚集性的影响 (Inhibitory Effect of Syringoylagmatine on Platelet Aggregation in Rabbit)" *安徽医药 (Anhui Medical and Pharmaceutical Journal),* Vol. 16, No. 12, 31 December 2012 (2012-12-31), abstract, section 2.2 | 1-6, 9, 11-15, 18-21, 25 |

☑ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 April 2021** | **22 April 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/103136**

**C. DOCUMENTS CONSIDERED TO BE RELEVANT**

| Category* | Citation of document, with indication, where appropriate, of the relevant passages | Relevant to claim No. |
|---|---|---|
| Y | 许勤龙 等 (XU, Qinlong et al.). "N-(4-胍基丁基)丁香酰胺对家兔血小板聚集性的影响 (Inhibitory Effect of Syringoylagmatine on Platelet Aggregation in Rabbit)" *安徽医药 (Anhui Medical and Pharmaceutical Journal),* Vol. 16, No. 12, 31 December 2012 (2012-12-31), abstract, section 2.2 | 7-8, 16-17, 23 |
| X | 张恩立 等 (ZHANG, Enyi et al.). "阿魏酰胍丁胺对家兔血小板聚集性的影响 (non-official translation: Effects of Feruloylagmatine on Platelet Aggregation in Rabbits)" *时珍国医国药 (Lishizhen Medicine and Materia Medica Research),* Vol. 24, No. 1, 31 December 2013 (2013-12-31), abstract, section 2.2 | 1-6, 9, 11-15, 18-21, 25 |
| Y | 张恩立 等 (ZHANG, Enyi et al.). "阿魏酰胍丁胺对家兔血小板聚集性的影响 (non-official translation: Effects of Feruloylagmatine on Platelet Aggregation in Rabbits)" *时珍国医国药 (Lishizhen Medicine and Materia Medica Research),* Vol. 24, No. 1, 31 December 2013 (2013-12-31), abstract, section 2.2 | 7-8, 16-17, 23 |
| X | 单春文 等 (SHAN, Chunwen et al.). "溴苄铵对豚鼠和家兔血小板聚集的影响 (non-official translation: Effects of Bretylium on Platelet Aggregation in Cavia Porcellus and Rabbits)" *第一军医大学学报 (Journal of First Military Medical University),* Vol. 4, No. 3, 31 December 1984 (1984-12-31), Result and Discussion Part | 7-8, 16-17, 23 |

Form PCT/ISA/210 (second sheet) (January 2015)